# FASCICULE DE BREVET EUROPEEN

(11) **EP 0 635 516 B1**
(45) Date de publication et mention de la délivrance du brevet: **03.11.1999**
(21) Numéro de dépôt: 94201064.6
(22) Date de dépôt: 17.07.1992
(51) Int. Cl.: C07H 15/04, B01F 17/00, C11D 1/66, A61K 7/08, C09K 19/06, A23L 1/03, C07H 7/033

(54) **Dérivés de l'acide galacturonique, leurs procédés de préparation et leurs applications**
Galakturonsäure-Derivate, Verfahren zur ihrer Herstellung und ihre Verwendung
Galacturonic acid derivatives, process for their préparation and their uses

(30) Priorité: 22.07.1991 FR 9109226
(43) Date de publication de la demande: 25.01.1995
(62) Demande divisionnaire de: 92402076.1
(73) Titulaire: AGRO INDUSTRIE RECHERCHES ET DEVELOPPEMENTS (A.R.D.), F-75008 Paris (FR); ZSCHIMMER & SCHWARZ GmbH & Co. CHEMISCHE FABRIKEN, D-56112 Lahnstein (DE)
(72) Inventeur: Petit, Serge, F-60 150 Montmach (FR); Ralainirina, Robert, F-80 080 Amiens (FR); Favre, Serge, F-60 200 Compiegne (FR); Baynast de, Régis, F-78000 Versailles (FR)
(74) Mandataire: Eidelsberg, Victor Albert

(56) Documents cités:
- EP-A- 0 326 673
- EP-A- 0 334 498
- EP-A- 0 427 210
- US-A- 3 873 614
- TENSIDE, SURFACTANTS, DETERGENTS, vol.26, 1989 pages 318 - 324 TH. BÖCKER ET AL 'Synthese und Eigenschaften von Kohlenhydrattensiden'
- CHEMICAL ABSTRACTS, vol. 109, no. 21, 21 Novembre 1988, Columbus, Ohio, US; abstract no. 190679t, Y. HUI ET AL. 'Synthesis of glucoside surfactants and asymmetric reduction of phenyl alkyl ketones in chiral micelles' & HUAXUE XUEBAO, vol.46, 1988 pages 239 - 245
- LIFE SCIENCES, vol.50, 1992 pages 1773 - 1779 Y. NAMBA ET AL 'Liposomal modification with uronate, which endows liposomes with long circulation in vivo, reduces the uptake of liposomes by J744 cells in vitro'
- Chemistry Letters (1989), vol. 2148, 2145-2148, Y. NAMBA et al.

## Description

La présente invention concerne des dérivés de l'acide galacturonique, leurs procédés de préparation et leurs diverses applications, notamment comme agents tensio-actifs non ioniques ou anioniques.

Le greffage de groupes alkyle sur des glucides conduit à des agents tensio-actifs dont les propriétés de surface sont très intéressantes et dont la biodégradabilité est généralement bonne (R.D. SWISHER, "Surfactant biodégradation", Marcel Dekker, Inc. NEW YORK, 1987).

Les matières premières le plus souvent utilisées sont le saccharose et le glucose et les réactions effectuées sur ces substrats non protégés conduisent à des mélanges complexes de tensio-actifs non ioniques, ceci étant dû à des greffages statistiques (mono, di, triaddition) et à des phénomènes d'oligomérisation inter et/ou intra moléculaires (voir EP 0249013, DE 3842541, DE 3723826, H. LUDERS et P. HOFFMANN "Synthesis, Chemical Structure and Properties of Alkylpolyglucosides", Césio, 2ème Congrès Mondial sur les Surfactants, 24-27 mai 1988, PARIS, et, D. BALZET "Alkylpolyglucosides, their physico-chemical properties and their uses", Tenside Surf. Det. 28. 1991, 6). Les températures de réaction utilisées dans ces préparations sont généralement élevées (supérieures à 100 °C), ce qui entraîne une dégradation partielle des substrats et provoque une coloration des produits.

Pour ce qui est des tensio-actifs anioniques à base de glucides, les rares composés décrits dans la littérature sont obtenus par bioconversion (sophoroses lipides, rhamnolipides... : voir DE 352 6417 ; G. Georgiu, S.C. Lin et M.M. Sharma, "Surface active compounds from microorganisms". Bio./Biotechnology, vol 10, January 1992. 60-65 : D.F. Gerson et J.E. Zajic "microbial biosurfactants, Process Biochemistry, July 1979, 20-29 ; D.G. Cooper "Biosurfactants", Microbiological Sciences", vol 3, n° 5, 1986, 145-149) ou par oxydation de l'hydroxyle primaire de D-octyl-glucopyranoside, de l'α-Ddodécylglucopyranoside, de l'α-D-tetradécyl glucopyranoside du β-D-décyl maltoside, de l'α-D-tetradécyl maltoside (voir EP 0326673 ; Bocker J. Thiem J., Tenside, Surf, Det., 26 318, (1989) ; Van Bekkum H. dans "Carbohydrates as organic raw materials" Lichtenthaler F.W. (Ed). V C.H. Weinheim 289 (1991) et références citées de GOEDE A.T.J.W., de Wit P. Vinki P. Van Rantwijk F. et Van Bekkum H, présentation au 6ème Congrès Européen sur la chimie des carbohydrates, Edimbourg, 1991), soit par voie catalytique, soit par voie chimique avec le complexe trioxyde de soufre/pyridine [voir Miam. H, Anderson C.E. et Kent P.W. Biochem. J., 181, 387,(1979)] pour obtenir respectivement les composés carboxylés ou sulfatés correspondants. Ces documents ne décrivent pas de dérivés du galactose, ni bien sur leurs propriétés, malgré des généralisations hasardeuses. De plus, les substrats nécessaires à ces synthèses sont difficiles à obtenir, et les seuls protocoles conduisant à des composés de structure bien définie nécessitent toujours une protection préalable, utilisent des solvants organiques et des réactifs coûteux (carbonate d'argent) ; c'est le cas notamment de l'octyl glucoside et de ses homologues (voir Rosavear P. van Aken T. Baxter J., Fergusson-Miller S., Biochemistry 19, 4108, 1980 ; Shimamoto T, Saito S., Tsuchiya T., J. Biochem., 97, 1807, 1985; Schmidt R.R., Angews Chem. Intern. Ed. Engl., 25, 212, 1986; Straathof A.J.T., Romein F., van Rantwijk F., Kieboom A.P.G. et van Bekkum H., Starch. 39 (10), 362, 1987).

Ces techniques ne satisfont pas à la demande actuelle de tensio-actifs non ioniques, (notamment formant un système bicaténaire), ou anioniques et pouvant être préparés à des prix bas et d'une efficacité plus grande que ceux utilisés jusqu'ici.

EP-A-0 326 673 décrit un procédé de préparation de tensioactifs mettant en oeuvre du galactose ou des oligomères de galactose onéreux et des catalyseurs onéreux et difficiles à recycler. L'article de Life Sciences 1992, vol. 50, pages 1773-9 décrit le palmityl-alpha-D-galacturonide préparé selon un procédé qui nécessite de nombreuses étapes, des solvants, des catalyseurs et des purifications difficiles à transposer à l'échelle industrielle (chromatographie). Tenside, Surfactants, Détergents Vol. 26, 1989, pages 318-324 et chemical abstract vol. 109 n° 21, 21 novembre 1988, Columbus Ohio, US abstract n° 190679t décrivent la synthèse coûteuse de tensioactifs à l'aide de catalyseurs.

Des tentatives récentes (D. Plusquellec et collaborateurs, Anal. Bioch. 179, 145 (1989); EP 334498 ; EP 427210 F. Bjorkling et collaborateurs, J. Chem. Soc., Chem. Commun, 934 (1989); K. Adelhorst et collaborateurs, Synthesis, 112-115 (1989); M.P. de Nijs et collaborateurs, Recl. Trav. Chim., Pays-Bas, 109, 429-433 (1990), permettent d'obtenir des agents tensio-actifs non ioniques de structure bien définie, et sans protection du substrat hydrophile. Toutefois, elles nécessitent une étape chimique de glycosilation et une étape enzymatique permettant le greffage d'un deuxième groupe alkyle sur la position alcool primaire du glucose. Les inconvénients majeurs d'une telle technique résident dans les limitations apportées par les lipases qui n'acceptent bien que cenains substrats glucosidiques et dont les rendements de greffage sont très variables en fonction de la longueur de l'acide ou de l'ester gras mis en réaction. Si, dans quelques cas très favorables, les rendements peuvent atteindre 60 %, ils sont de façon plus générale inférieurs à 20 %. De plus, le coût du traitement enzymatique est loin d'être négligeable et la préparation d'un substrat alkylglycoside de bonne pureté n'est pas une opération facile.

Par ailleurs, les composés décrits, notamment dans EP 427210 (Lion Corporation) et EP 334498 (Cerestar Holding), différent de ceux de la présente invention puisque obtenus par esterification (enzymatique) par un acide gras de 13 position hydroxyle primaire d'alkyl glucosides. La généralisation, d'ailleurs hasardeuse (vu la spécificité de la catalyse enzymatique), de cette technique à l'ensemble des hexoses ne conduirait pas, même en série "galactose", aux composés qui font l'objet de l'invention.

En effet, les composés bicaténaires qu'obtiendraient ces auteurs en série "galactose" seraient de structure générale suivante : 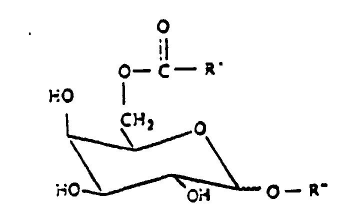 alors que les isomères "pyranose" de la présente invention sont de structure générale :

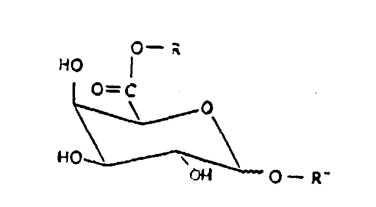

L'invention vise des dérivés de l'acide galacturonique qui peuvent être préparés sans phénomène d'oligomérisation, par voie purement chimique, sans protection préalable mais avec une régiosélectivité parfaite et des rendements élevés et qui s'avèrent doués de propriétés, notamment tensio-actives, supérieures à celles des meilleurs tensio-actifs utilisés jusqu'ici.

L'invention a pour objet des dérivés de l'acide galacturonique de formule :

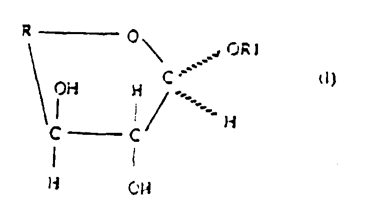

R1 étant alkyle linéaire ou ramifié ayant de 8 à 22 atomes de carbone,

R étant :

〉CH―CH(OH)―CO₂H₂

OU 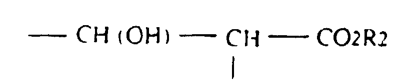 dont le carbone ponant le groupe hydroxyle n'est pas relié à l'atome d'oxygène endocyclique.

R2 étant l'hydrogène, un atome de métal alcalin, de métal alcalino-terreux ou un groupe ammonium quaternaire de formule: 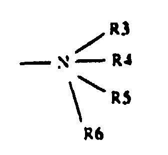 dans lequel chacun à R3 à R6
est indépendamment des autres l'hydrogène, alkyle ayant de 1 à 6 atomes de carbone, ou hydroxyalkyle ayant de 1 à 6 atomes de carbone, à l'exception du palmityl-alpha-D-galacturonide.

Le métal alcalin en notamment le sodium, le potassium, et le métal alcalino-terreux, le magnésium. Comme sels d'ammoniums quaternaires, on peut citer ceux de méthyl-2, hydroxyméthyl-2 éthyl ammonium, de tri (hydroxyméthyl) méthyl ammonium, de (dihy droxyméthyl)-2, 2-éthyl ammonium.

Parmi ces dérivés, on préfère pour leur bon pouvoir moussant ceux dans lesquels R1 est alkyle ayant de 8 à 14 atomes de carbone notamment les sels de l'acide dodécyle galactoside uronique dont le pouvoir moussant est supérieur aux produits classiques de référence. L'évaluation est réalisée selon la norme NFT 73404, qui consiste à faire s'écouler, selon un débit constant, 500 ml de solution de tensio-actif dans une éprouvette de 1000 ml graduée et thermostatée, contenant 50 ml de la même solution. La quantité de mousse, générée par l'écoulement, est estimée volumétriquement juste après la fin de l'écoulement (le paramètre mesuré est un volume initial de mousse).

La stabilité de la mousse est également prise en compte par mesure du volume durant 20 minutes.

Le dodécyl D-galactoside uronate de sodium est plus efficace que le dodécyl benzène sulfonate de sodium (SDBS), le dodécyl sulfate de sodium (SDS) ou le lauryl éther sulfate de sodium à 2 moles d'oxyde d'éthylène (LES) , son volume initial de mousse est de 520 ml alors qu'il est inférieur ou égal à 450 ml pour les autres.

Leur pouvoir moussant est sensiblement égal à celui des alkylpolyglucosides (APG) dont les coûts, en raison de leur procédé de fabrication et de purification (lié notamment à des problèmes de coloration et de stabilité en milieu basique du produit), dépassent le double de ceux des dérivés de l'invention.

Par ailleurs, ces composés présentent une très bonne stabilité dans le temps de la mousse, puisque le pourcentage de perte est inférieur à 5% après 20 minutes.

Les sels de l'acide octyl galactoside uronique et décyl galactoside uronique ont également de bons pouvoirs mouillants qui sont supérieurs à ceux de lauryl éther sulfate de sodium à 2 moles d'oxyde d'éthylène (LES), du dodécyl sulfonate de sodium (SDS) et des alkyl polyglucosides (APG) après 400 secondes. Le test effectué consiste à suivre durant 600 secondes, la quantité de solution de tensio-actif absorbée par un tissu de coton. La pièce de tissu vient effleurer la surface de la solution de tensio-actif et la traction générée par la montée capillaire de la solution est enregistrée en continu durant 600 secondes.

Le tissu utilisé est un coton écru découpé en pièces de (2cm sur 2 cm, soit environ 0,12 g) et répondant aux spécifications de la norme NFT 73406. L'appareil utilisé est un tensiomètre KRUSS automatique, muni du logiciel d'adsorption K121. Les mesures sont effectuées à 25 °C.

Le détergent suivant l'invention imprègne ainsi mieux le linge à laver, au cours d'une lessive de plusieurs dizaines de minutes, que les détergents antérieurs.

La mesure des angles de mouillage est effectuée à l'aide du tensiomètre KRUSS muni du logiciel "K121 Contact Angle" contre une plaque de polyéthylène de 2 cm de longueur.

Ce logiciel permet l'enregistrement en continu du poids du polyéthylène durant sa pénétration de 5 mn dans la solution de tensio-actif. L'angle de contact à l'avancée est déterminé par extrapolation mathématique à l'origine.

Les résultats confirment l'intérêt des alkyl galactoside uronates dans le domaine de la détergence, notamment pour les chaînes alkyle ayant jusqu'à 14 atomes de carbone. On préférera le décyl galactoside uronate de sodium dont l'angle de mouillage (3°) est largement inférieur à celui du laury'l sulfate de sodium (70°), du lauryl éther sulfate de sodium à deux moles d'oxyde d'éthylène (60°) et du dodécyl benzène sulfonate de sodium (38°). Là encore, ces valeurs montrent que le détergent selon l'invention imprègne mieux le linge à laver que les détergents antérieurs.

Les alkyl galactoside uronates préparés abaissent très efficacement la tension superficielle de l'eau. Cette propriété a été déterminée par une technique usuelle de tensiométrie, en utilisant un tensiomètre KRUSS de type K12, et selon la norme ISO 304. La mesure est effectuée à 25 °C. Le mobile de mesure est une lame de platine rectangulaire (25 mm x 5 mm).

L'appareillage entièrement automatique permet d'effectuer des mesures répétitives et de calculer la moyenne statistique de 10 valeurs.

Quand R1 a moins de 12 atomes de carbone, cet abaissement de tension superficielle est supérieur à celui observé avec les produits connus (Lauryl sulfate de sodium, lauryl éther sulfate de sodium à 2 mole d'oxyde d'éthylène, sodium dodécyl benzène sulfonate de sodium, alkyl glucoside, alkyl polyglucosides...) ; il est équivalent quand R1 a plus de 12 atomes de carbone. La mise en émulsion des salissures portées par du linge est ainsi rendue plus facile.

L'ensemble de ces propriétés (pouvoir moussant, mouillabilité, angle de mouillage, abaissement de la tension superficielle...) font que les alkyl galactoside uronates peuvent notamment être utilisés en détergence (domaine lessiviel notamment). On préfèrera les alkyl galactoside uronates de sodium, notamment, ayant de 8 à 14, particulièrement 8 à 12, et plus particulièrement 10 atomes de carbone dans leur chaîne alkyle.

L'invention a également pour objet un procédé pour donner de la tensio-activité à une composition caractérisée en ce qu'il consiste à lui incorporer de 0,1 à 60 % en poids d'un dérivé ou d'un mélange de dérivés suivant l'invention.

Une composition détergente en poudre suivant l'invention comprend de 0,1 à 60 %, et de préférence de 10 à 30 % en poids d'une base détergente et de 99,9 à 40 %, et de préférence de 90 à 70 % en poids d'adjuvants.

La base détergente peut être un dérivé ou un mélange de dérivés selon l'invention. Elle peut également être un mélange de un ou plusieurs dérivés selon l'invention avec un ou plusieurs tensio-actifs classiques dans le domaine ; ces tensio-actifs pouvant être anioniques, non ioniques, cationiques ou amphotères. La proportion de tensio-actifs selon l'invention représente de 1 à 100 %, en poids, et de préférence de 50 à 100 % de l'ensemble de la charge en tensio-actifs.

Les tensio-actifs anioniques de la composition, autres que ceux de l'invention, peuvent être des alkylbenzène sulfonates, des sulfates d'alcools gras, des éthers sulfates d'alcools gras, des α-oléfines sulfonates et parmi ceux relatifs à l'invention seront préférées ceux avec R1 étant alkyle ayant de 8 à 14 atomes de carbone. L'ensemble des tensio-actifs anioniques de la composition représentent de 30 % à 90 % en poids, et de préférence de 40 à 70 % en poids de l'ensemble de la charge en tensio-actifs. Les tensio-actifs non ioniques de la composition, quand ils sont autres que ceux de l'invention, peuvent notamment être des éthers d'alkyl poly (éthylène glycol), des éthers de nonylphénylpoly (éthylène glycol).

Les adjuvants sont les "builders", les agents de blanchiement, et divers additifs tels les agents anti-redéposition, les agents anti-corrosion, des enzymes, des azurants optiques, des exhausteurs ou des régulateurs de mousse, des colorants, des parfums, des opacifiants...

Les builders peuvent être un phosphate, notamment un triphosphate, par exemple de métal alcalin, et en particulier de sodium, l'acide nitrilotriacétique ou ses sels de métaux alcalins, notamment de sodium, l'acide citrique ou ses sels, notamment de métaux alcalins, notamment de sodium, un acide glyconique, notamment l'acide gluconique ou l'acide galactonique, et ses sels, notamment de métal alcalin, et en particulier de sodium, un carbonate de métal alcalin, notamment de sodium, un acide polyacrylique ou ses sels, de métal alcalin notamment.

On peut utiliser ces builders en mélange selon différentes proportions. Le rapport de la charge de l'ensemble des builders sur celle de l'ensemble de la base tensio-active est compris entre 1 et 4, et de préférence entre 2 et 3.

Les agents de blanchiement peuvent être un perborate, de métal alcalin notamment, et en particulier de sodium, un percarbonate, de métal alcalin notamment, et en particulier de sodium, et peuvent contenir ou non un activateur de blanchiement, notamment le tétraacétylglycolurile, le 1,5-diacétyl 2,4-dioxo-hexahydro-1,3,5-triazine, le tétraacétyl éthylène diamine, la N,N-diacétyl N,N'-diméthylurée, les polyacétates de carbohydrates, en particulier d'hexoses ou de pentoses, et plus particulièrement de glucose, ou de saccharose, ou un stabilisateur de blanchiement, choisi parmi les stabilisateurs usuels, notamment le tétraacétate d'éthylène diamine (EDTA) ou des phosphonates. Ces agents de blanchiement représentent de 0,2 à 25 % en poids de la composition détergente en poudre.

Les agents anti-redéposition peuvent être des éthers de cellulose, notamment de carboxyméthyl cellulose, et leurs sels de métaux alcalins, notamment de sodium, ou des polymères synthétiques usuels dans ce type de formulation. Ils représentent de 0,5 à 3 %, et de préférence de 0,5 à 2 % en poids de la composition détergente. L'agent anticorrosion peut être un silicate de métal alcalin, notamment de sodium, dont la proportion représente 0,5 à 25 % en poids de la composition détergente.

Les enzymes sont notamment des protéases ou des amylases. Les azurants optiques sont ceux utilisés classiquement dans le domaine, notamment l'acide stilbène disulfonique ou les dérivés du bis (styryl)biphényl. Les exhausteurs de mousse peuvent être alkyl éthanolamide, notamment le cocomonoéthanolamide. Les régulateurs de mousse peuvent notamment être des silicones, des savons ou des paraffines.

La composition détergente en poudre est utilisée à une concentration de 1 à 20 g/l, et de préférence entre 1 et 6 g/l. Le lavage est effectué dans une machine conventionnelle, entre 20 et 80 °C, et de préférence entre 20 et 60 °C, durant une période de 10 à 60 minutes.

Une composition détergente liquide suivant l'invention comprend de 0,1 à 60 %, et de préférence de 10 à 60 % en poids d'une base détergente de 99,9 à 40 % et de préférence de 90 à 70 % en poids d'adjuvants.

La base détergente peut être un dérivé ou un mélange de dérivés selon l'invention. Elle peut également être un mélange de un ou plusieurs dérivés selon l'invention avec un ou plusieurs tensio-actifs classiques dans le domaine ; ces tensio-actifs pouvant être anioniques non ioniques, cationiques ou amphotères. La proposition de tensio-actifs selon l'invention représente de 1 à 100 % en poids, et de préférence de 50 à 100 %, de l'ensemble de la charge en tensio-actifs. Les tensio-actifs anionique utilisés, quand ils sont autres que ceux de l'invention, peuvent notamment être un alkyl benzène sulfonate, un savon ou un éther sulfate d'alcool gras. Les tensio-actifs non ioniques utilisés, quand ils sont autres que ceux de l'invention peuvent notamment être des éthers d'alkyl poly (éthylène glycol). Lorsqu'un tensio-actif cationique est incorporé à la formulation il ce peut être un chlorure de dialkyldiméthyl ammonium.

Les ingrédients classiques complètent la formulation ; il s'agit notamment d'exhausteurs de mousse (0-2 %), d'enzymes, notamment de protéases (0 - 2 %) de builders, (0-30 % et de préférence de 10 à 30 %) notamment le citrate de sodium, le silicate de sodium, des zéolites ou de polycarboxylates, des stabilisants, notamment la tri et la monoétanolmine et des agents chelatants, des solvants de solubilisation (5-15 %), notamment l'éthanol ou le propylène glycol, (5-15 %), des azurants optiques, des argiles, des parfums, des colorants et de l'eau (30 - 60 %).

L'invention a également pour objet l'utilisation des dérivés selon l'invention pour obtenir une composition détergente pour le lavage de la vaisselle, et pour les usages ménagers. Cette composition comprend de 1 à 30 % en poids et de préférence de 5 à 25 %, de dérivés selon l'invention et 99,9 à 70 % en poids, et de préférence 95 à 75 % d'adjuvants. Ces adjuvants peuvent être d'autres tensio-actifs anioniques, notamment un sulfate d'alcool gras éthoxylé (0-20 % en poids), un épaississant, notamment le chlorure de sodium (0-5 %), un mono ou triéthanolamide d'acide gras éthoxylé ou non (0-15 %) un polyacrylate de sodium (0-5 % en poids) un complexant du calcium, notamment l'EDTA (0-5 %), un solvant notamment l'éthanol (0-5 %) un oxyde d'amine grasse (0-10 %) un parfum, un colorant, un conservateur... en quantités suffisantes.

Les compositions selon l'invention sont agréables au toucher et se rincent facilement.

La composition détergente liquide selon l'invention est utilisée en solution aqueuse à une concentration de 6 à 12 g/l, et à des températures de 40 à 70 °C.

L'invention a aussi pour objet des compositions cosmétologiques comprenant de 0,1 à 50 % et de préférence de 5 à 35 % en poids de substance active et de 99,9 à 50 % et de préférence de 95 à 65 % en poids d'excipients, caractérisé en ce que le détergent ou l'adoucissant est un dérivé de formule I selon l'invention. On préférera, pour leur faible concentration micellaire critique (0,3 à 1,2 g/l), l'octadécyl, l'hexadécyl et le tetradécyl galactoside uronates en raison de l'agressivité plus faible vis à vis des muqueuses et de la peau (Lang G. et Spengler I., Prépurits. IF SCC Congr., 1986, vol I p25). On préférera, pour leur bon pouvoir moussant, les décyl et dodécyl galactoside uronates de sodium notamment.

La composition cosmétologique peut être un savon liquide doux, contenant 5 à 30 % en poids, et de préférence 5 à 20 %, d'un dérivé selon l'invention et 95 à 70 % en poids, et de préférence 95 à 80 % en poids d'excipients. Ces excipients peuvent être un autre tensio-actif anionique notamment le cocoyl isethionate de sodium (0-10 % en poids), le lauryl sulfate de Na (0-10 %) le sel de sodium d'un alkyl peptide (0-15 % en poids), un tensio-actif amphotère, notamment un alkyl amidopropylbétaine tel le cocoamidopropyl bétaine (0-10 % en poids), une huile minérale lourde (0-20 % en poids), un dérivé cellulosique, notamment un carboxyméthyl cellulose (0-1 %), un solvant notamment un alcool tel l'éthanol ou le propylène glycol (0-5 % en poids), un complexant notamment l'EDTA (0-2 %), du chlorure de sodium (q.s), un alcool gras, notamment l'alcool cétylique (0-5 % en poids), des conservateurs, des parfums, des colorants (qs).

La composition cosmétologique peut être un shampooing, notamment un shampooing doux à usage fréquent. Il est composé de 5 à 35 % en poids d'une base détergente dont, de préférence, 10 à 75 % est constitué par un dérivé ou un mélange de dérivés selon l'invention, et de 95 à 65 % d'adjuvants.

Les autres tensio-actifs constituant la base détergente peuvent être un alkyl éther sulfate tel le lauryl éther sulfate de sodium ou de magnésium, un alkyl éther sulfate de sodium polyoxyéthylèné, tel le lauryl éther sulfate de sodium polyoxyéthyléné, une alkyl bétaine telle la cocoylbétaine, une alkyl amidopropyl bétaine telle la cocoyl amidopropyl bétaine, une alkyl diméthylamino acétique acide bétaine telle la lauryl diméthylamino acétique acide bétaine, une alkyl diméthylamino hydroxypropyl sulfobétaine un α-oléfine sulfonate de sodium, un alkyl polyéthylène glycol tel l'octadécyl PEG 15, une alkyl imidazolinium bétaine telle la cocoyl imidazolinium bétaine, un alkyl éther sulfosuccinate tel le lauryl éther sulfo succinate de disodium, un β alkyl amino propionate tel le sodium β-laurylaminopropionate, un alkyl diamino éthyl glycine tel le sodium lauryl diaminoéthylglycine et, lorsqu'ils sont des dérivés selon l'invention, ils sont de préférence des alkyls galactoside uronates avec alkyl ayant de 10 à 14 atomes de carbone.

Les adjuvants peuvent être des épaississants, des texturants, tels des diéthanolamide d'acides gras, notamment le cocoyl diéthanolamide tel un alkyl acrylate, notamment le laurylacrylate, le chlorure de sodium, un dialkylcarboxylate d'éthylène glycol tel le distéarate d'éthylène glycol, un N-oxyde d'amine grasse tel le N-cocoyl oxyde..., qui sont incorporés à hauteur de 0-10 % en poids dans la formulation. Les adjuvants peuvent être également des agents de conditionnement, des adoucissants tels des hydrolysats de protéines de blé, tels des éthers de cellulose contenant des ammoniums quaternaires (teneur en azote 1-3 %, PM = 50-150 000), un copolymère acrylamide /chlorure de diméthyl alkyl ammonium, représentant de 0,5 à 5 % en poids de la formulation, des complexants, notamment l'EDTA ou l'acide galactarique représentant 0,1 à 1 % en poids de la formulation, et finalement des parfums, des agents nacrants, des conservateurs, des acidifiants en quantité suffisante, et de l'eau purifiée.

La composition cosmétologique peut être un bain moussant contenant de 5 à 35 % d'une base détergente, elle même constituée de plus de 50 % de dérivés ou mélange de dérivés selon l'invention, et d'adjuvants. Les autres constituants de la base détergente sont les composés classiques dans le domaine et peuvent notamment être des alkyl amido bétaines, tel le cocoylamidopropyl bétaine, un alkyl carboxylate de sorbitan éthoxylé, tel le laurate de sorbitan éthoxylé.

Les adjuvants sont les mono ou tri éthanolamide d'acides gras (0-10 % en poids), un dialkylcarboxylate de propylène glycol éthoxylé (0-5 % en poids), un polyéthylène glycol, notamment le triéthylène glycol (0-5 % en poids), un alkyl acrylique, notamment un oleyl acrylique (0-5 % en poids), une huile végétale, notamment l'huile d'amandes douces (0-10 % en poids), du chlorure de sodium (qs), de l'EDTA (0-0,5 % en poids), un alcool gras notamment l'hexadécanol (0-2 % en poids), un conservateur, un parfum, un colorant en quantité suffisante et de l'eau.

La composition cosmétologique peut être un gel douche contenant 5 à 35 % d'une base détergente, elle-même constituée d'au moins 50 % de dérivés, ou mélange de dérivés suivant l'invention et d'adjuvants.

Les tensio-actifs pouvant compléter la base détergente lorsqu'ils ne sont pas ceux de l'invention, peuvent être un alkyl sulfosuccinate polyoxyéthyléné, tel le cocoyl sulfo succinate à 3 moles d'oxyde d'éthylène, un alkyl amido-N-glycinate tel le C12-C18 amido-N-glycinate, un alkyl sulfate d'ammonium, tel le lauryl sulfate d'ammonium

Les adjuvants peuvent être un alkylcarboxylate de propylène glycol éthoxylé tel le dioléate de propylène glycol éthoxylé (0-5 % en poids), une alkyl amido bétaine tel un lauryl amidopropyl bétaine (0-5 % en poids), un agent nacrant (0-7 % en poids), un gel acrylique, (0-1 % en poids), du chlorure de sodium, un complexant, un conservateur, un parfum, en quantités suffisantes et de l'eau purifiée.

La composition cosmétologique peut également être une composition de type dentifrice, un bain pour l'hygiène buccale, un syndet....
La composition cosmétologique peut, notamment par incorporation d'alkyl galactosides uronate d'alkyles, d'octyl galactoside uronate d'octyl notamment qui forme une structure de type gel pour des concentrations en eau supérieures à 60 %, et de préférence supérieures à 90 %, être une crème de soins, pour le visage notamment.

Les dérivés selon l'invention sont également des cristaux liquides extrinsèques, lorsqu'ils sont à des concentrations comprises entre 5 et 60 % en poids, dans l'eau notamment.

On peut préparer les composés suivant l'invention par un procédé qui consiste à faire réagir un alcool de formule RIOH sur l'acide galacturonique de formule : 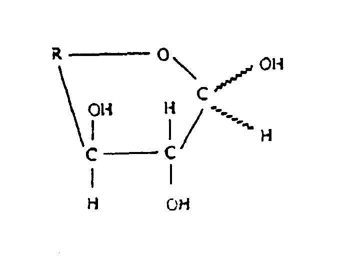 R étant

〉CH―CH(OH)―CO₂H

ou 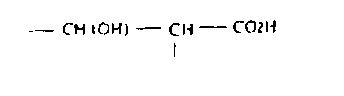 dont le carbone ponant le groupe hydroxyle n'est pas relié à l'atome d'oxygène endocyclique pour obtenir un mélange d'alkylgalactoside uronates d'alkyles de formule 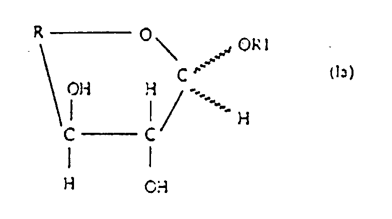
dans laquelle R2 est R1 et R a les deux significations indiquées et à séparer chaque alkylgalactoside uronate d'alkyle du mélange,
et pour obtenir les dérivés (I) dans lesquels R2 est un métal alcalin, un métal alcalino-terreux ou un groupe ammonium quaternaire, à salifier un dérivé de formule (Ia) par une base de formule Me (OH)x dans laquelle Me est un métal alcalin, un métal alcalino-terreux ou 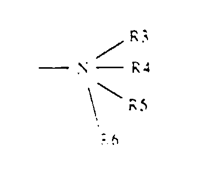 x étant la valence du métal ou étant égal à 1 quand Me est 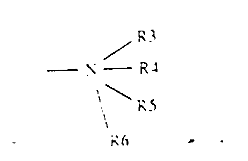 et pour obtenir les dérivés (I) dans lesquels R2 est l'hydrogène, à acidifier les dérivés (I) dans lesquels R2 est un métal ou un groupe ammonium quaternaire par un acide.

L'acide galacturonique peut être préparé par hydrolyse de pectines (Anderson King J., J. Chem. Soc., 1961, page 5333).

A la différence de l'art antérieur, qui nécessite la réaction d'un acide ou d'un ester inférieur sur un sucre présentant un certain nombre d'hydroxyles sur chacun desquels l'acide ou l'ester réagit avec formation d'autant de chaînes latérales, le procédé suivant l'invention met en jeu la réaction d'un alcool sur un sucre possédant, outre une fonction alcool anomérique privilégiée, à savoir la fonction alcool portée par le carbone en alpha de l'oxygène endocyclique, une fonction acide carboxylique, en sorte que la réaction de l'alcool a lieu exclusivement sur ces deux positions, ce qui donne une régiosélectivité parfaite avec seulement deux chaînes latérales en des positions bien définies.

En outre, comme la réaction d'estérification (sur la fonction acide carboxylique) est plus rapide que celle de glycosidation (sur la fonction alcool anomère), le sucre de départ ou substrat, initialement peu soluble dans le milieu réactionnel constitué d'alcool additionné éventuellement d'un solvant, prend rapidement un caractère lipophile qui le rend plus soluble et favorise la réaction de glycosidation, qui peut dès lors se dérouler plus rapidement dans des conditions plus douces, ce qui se traduit par une moindre coloration du produit obtenu, tout en ayant un grand rendement.

### En pratique le procédé consiste :

Pour préparer les alkyl galactoside uronates d'alkyle :
- à mettre en contact un équivalent d'acide galacturonique;
- de 2 à 50 équivalents molaires, et de préférence de 2 à 10 équivalents molaires d'un alcool de formule R1 OH;
- de 10⁻³ à 1, et de préférence de 10⁻² à 10⁻¹ équivalent molaire d'un catalyseur acide, tel que l'acide chlorhydrique, l'acide sulfurique, un acide alkyl sulfurique tel l'acide décyl ou lauryl sulfurique, un acide sulfonique tel l'acide benzènesulfonique, l'acide paratoluène sulfonique, l'acide camphresulfonique, un acide alkylsulfonique tel l'acide méthylsulfonique, l'acide décylsulfonique, l'acide laurylsulfonique, l'acide sulfosuccinique ou un sulfosuccinate d'alkyl tel le sulfosuccinate de décyle ou sulfosuccinate de lauryle, les acides per halohydriques, tel que l'acide perchlorique, des métaux tels que le cuivre ou le fer, leurs oxydes ou leurs sels, comme leurs halogénures, des halogènes, comme l'iode, des pentahalogénures d'antimoine, notamment des pentachlorures ou pentafluorures, ou des sulfates de titane.

Cette catalyse acide peut également être effectuée par 0,05 à 6 équivalents pondéraux d'une résine sulfonique sous sa forme H^{+,} ou d'une argile acide. Lorsque l'effet deshydratant de la résine est utilisé, on préfère pour cette catalyse hétérogène les résines à forte capacité de rétention d'eau.

On préfère l'acide sulfurique, un acide alkyl sulfurique, l'acide méthane sulfonique, un acide alkyl sulfonique, l'acide succinique ou un succinate d'alkyl, l'iode ou une résine sulfonique.
- De 2 à 20 équivalents en poids par rapport au substrat d'un solvant pouvant être un étheroxyde, tel le tétrahydrofuranne, le dioxanne, l'éther diméthylique de l'éthylèneglycol, l'éther diméthylique du diéthylèneglycol, un hydrocarbure halogéné tel le dichlorométhane, le chloroforme, le dichloroéthane, un ester tel que l'acétate d'éthyle, l'acétate de propyle ou l'acétate de butyle, un solvant nitré tel que le nitrométhane, le nitroéthane, le nitro-2- propane, un solvant de la famille des amides tel que le N-méthylformamide, le N,N-diméthylformamide, le N,N-diméthylacétamide, la N-méthyl-2-pyrrolidone, un nitrile tel que l'acétonitrile ou un alcane, de préférence l'hexane, l'heptane ou l'octane, ou un solvant aromatique tel que le toluène ou le xylène.

On peut également utiliser un mélange de deux ou plusieurs de ces solvants ou effectuer la réaction en l'absence totale de solvant.

Un agent de deshydratation classique, tel des tamis moléculaires ou des zéolites, qu'ils soient rajoutés directement dans le milieu réactionnel ou qu'on fasse circuler le filtrat de réaction à travers une colonne thermostatée emplie de cet agent de déshydratation peut être utilisé.
- A effectuer la réaction à des températures comprises entre 25 et 140 °C et, de préférence, entre 50 °C et 90 °C et pendant une durée de 1 heure à 3 jours et, de préférence, de 3 heures à 24 heures ;
- A effectuer la réaction sous une pression comprise entre 0,1 et 760 mm Hg, et de préférence entre 0,1 mm et 300 mm Hg ;
- A filtrer le catalyseur acide, lors d'une catalyse hétérogène ou à neutraliser le catalyseur acide, puis à filtrer son sel lors d'une catalyse homogène. La neutralisation du milieu réactionnel est faite par exemple par un hydrogénocarbonate de métal alcalin, notamment l'hydrogénocarbonate de sodium.
- A chromatographier sur colonne de silice le mélange de ces alkyles galactosides uronates d'alkyle pour les séparer.

### Pour préparer les sels des acides alkyl galactoside uroniques :

- à mettre en contact un équivalent d'alkyl galactoside uronates d'alkyles,
- de 0,1 à 20 équivalents en poids d'un solvant pouvant être l'eau, un alcane, de préférence le pentane, l'hexane, l'heptane ou l'octane, un étheroxyde, tel le tetrahydrofuranne, le dioxanne, l'éther diméthylique de l'éthylène glycol, l'éther diméthylique du diéthylène glycol, un hydrocarbure halogéné tel le dichlorométhane, le chloroforme, le dichloroéthane, un alcool, pouvant notamment être un alcool à chaîne carbonée courte, te! que le méthanol, l'éthanol, le propanol, l'isopropanol, le butanol, ou à chaine carbonée plus longue notamment tel que l'octanol, le décanol, le dodécanol, le tétradécanol, etc...

On peut également utiliser un mélange de deux ou plusieurs de ces solvants, ou effectuer la réaction en l'absence totale de solvant.

On préfère l'eau, le méthanol, l'éthanol, l'isopropanol, le tétrahydrofuranne, le pentane, l'hexane et l'heptane.
- de 0,5 à 10, et de préférence de 1 à 3 équivalents d'une base de formule 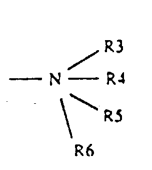 Me (OH)x dans laquelle Me est un métal alcalin, un métal alcalino-terreux ou cf formule P14 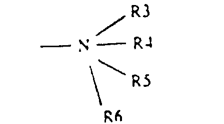 x étant la valeur du métal ou égal à 1 quand Me est (formule P14)

On préfère l'hydroxyde de sodium, l'hydroxyde de potassium, l'ammoniaque ou un hydroxyde d'alkyl (hydroxyalkyl) ammonium.
- Eventuellement, de 10⁻¹ à 10⁻³, et de préfèrence de 10⁻¹ à 10⁻², d'un catalyseur de transfert de phase, par exemple un agent tensio-actif non ionique, cationique ou anionique, parmi lesquels on préfère tout particulièrement le chlorure d'hexadécyl triméthylammonium. On peut également utiliser le chlorure de tétrabutyl ammonium, le chlorure de benzyl triméthyl ammonium, l'oléylamine, le chlorure de triactyl méthyl ammonium, le chlorure de tricaprylméthyl ammonium, le sulfate acide de tétrabutylammonium et, outre ces ions ammonium et amine, des ions phosphonium, des ethers oxyde couronne, des alcools ethoxylés, des cryptands, des amino polyéthers, des phosphorylsulfoxides, cenains ionophores naturels, des esters de glycerol, des esters de sorbitan, etc...

La réaction est de préfèrence effectuée sans catalyseur de transfert de phase.
- A effectuer la réaction à des températures comprises entre 0 °C et 100°C, et de préférence entre 0 °C et 60 °C et pendant une durée de 15 mn à 360 mn, et de préférencc de 15 mn à 120 mn.
- A éliminer par filtration ou par évaporation l'alcool R1OH, le solvant ou système de solvant utilisé.
- A récupérer, laver avec un solvant ou un mélange de solvant choisi dans la liste des solvants de réaction, puis sécher les sels des acides alkyl galactoside uroniques obtenus.
- A éventuellement reprendre ces sels d'acides alkyl galactoside uroniques avec 0,5 à 50, et de préférence 1 à 5 équivalents d'eau, et à décolorer la solution obtenue sur du charbon actif, ou sur une résine adsorbante.
- A filtrer le charbon actif ou la résine pour récupérer les sels des acides alkyl galactoside uroniques décolorés.

### Et pour préparer les acides alkyl galactoside uroniques :

A acidifier les sels des acides alkyl galactoside uroniques par un équivalent ou plus d'un acide tel que l'acide chlorhydrique, l'acide sulfurique, un acide sulfonique, une résine sulfonique sous sa forme H⁺.

Les exemples suivants illustrent l'invention :

### 1) Synthèse n° 1

### Préparation de n-octyl D-galactoside uronate de n-octyle (R1 : R2: n-octyle)

A 50 g (0,235 mole) d'acide D-galacturonique monohydraté, on ajoute 600 ml (494 g; 3,80 moles) de n-octyle et 30 g de résine S 100 de chez Bayer (type gel). On porte à 80 °C, et après 30 heures de réaction, on filtre la résine, on traite le filtrat au charbon actif, et on concentre sous le vide de la pompe à palettes (température de 73 °C et vide de 145 mPa) pour obtenir 90,7 g (92% de rendement) du n-octyl D-galactoside uronate de n-octyle sous forme d'une huile orangée. Une chromatographie flash effectuée sur du produit brut sur une colonne de silice (35 à 70 µm), en utilisant l'acétate d'éthyle comme phase mobile, permet d'isoler ses quatre formes isomères, dont les caractéristiques physico-chimiques sont données ci-dessous.
- Chromatographie "Flash" du produit brut obtenu sur colonne de silice (35 à 70 µm) en utilisant dans un premier temps les systèmes éluant acétate d'éthyle/hexane - 70/30 en volume pour séparer les forme β-furanose et α-furanose, puis de l'acétate d'éthyle pour séparer les forme β-pyranose puis α-pyranose.

| COMPOSE | Rf |
|---|---|
| α-furanose | 0,72 |
| β-furanose | 0,66 |
| β-pyranose | 0,52 |
| α-pyranose | 0,42 |

RMN¹³ C dans CDCl₃ :

| | C1 | C2 | C3 | C4 | C5 | C6 |
|---|---|---|---|---|---|---|
| β-furanose | 107,95 | 80,30 | 77,16 | 85,31 | 69,58 | 171,97 |
| α-furanose | 100,93 | 77,79 | 74,58 | 82,83 | 69,82 | 167,51 |
| β-pyranose | 102,90 | 70,68 | 73,09 | 69,91 | 74,01 | 167,60 |
| α-pyranose | 98,96 | 68,18 | 69,87 | 70,30 | 70,42 | 167,25 |

### 2) Synthèse n° 2

### Préparation du n-décyl-D-galactoside de n-décyle (I/R1 = R2 = n-décyle).

A 200 g (0,940 mole) d'acide D-galacturonique monohydraté, on ajoute 1000 ml (829 g ; 5,237 moles) de n-décyle et 5 g (26,28 mmoles) d'acide paratoluène sulfonique monohydraté. On porte à 60 °C. sous un vide de 260 mmg de mercure pendant 24 heures. Après distillation de l'excès de n-décanol, on récupère 473 g une huile limpide jaunâtre contenant environ 50 g de n-décanol résiduel. Rendement en n-décyle galactoside uronate de n-décyle 473 g (95 %).

Une chromatographie sur colonne de gel de silice (35 à 70 µm) en utilisant comme éluant un système éther de pétrole/ éther éthylique (1/1, V/V), puis de l'éther éthylique, et enfin un système éther éthylique/acétate d'éthyle (6/4 - V/V), permet de séparer les 4 isomères formés. Les isomères α pyranose, β pyranose et β furanose cristallisent après évaporation du solvant d'élution. Ils sont ensuite recristallisés.

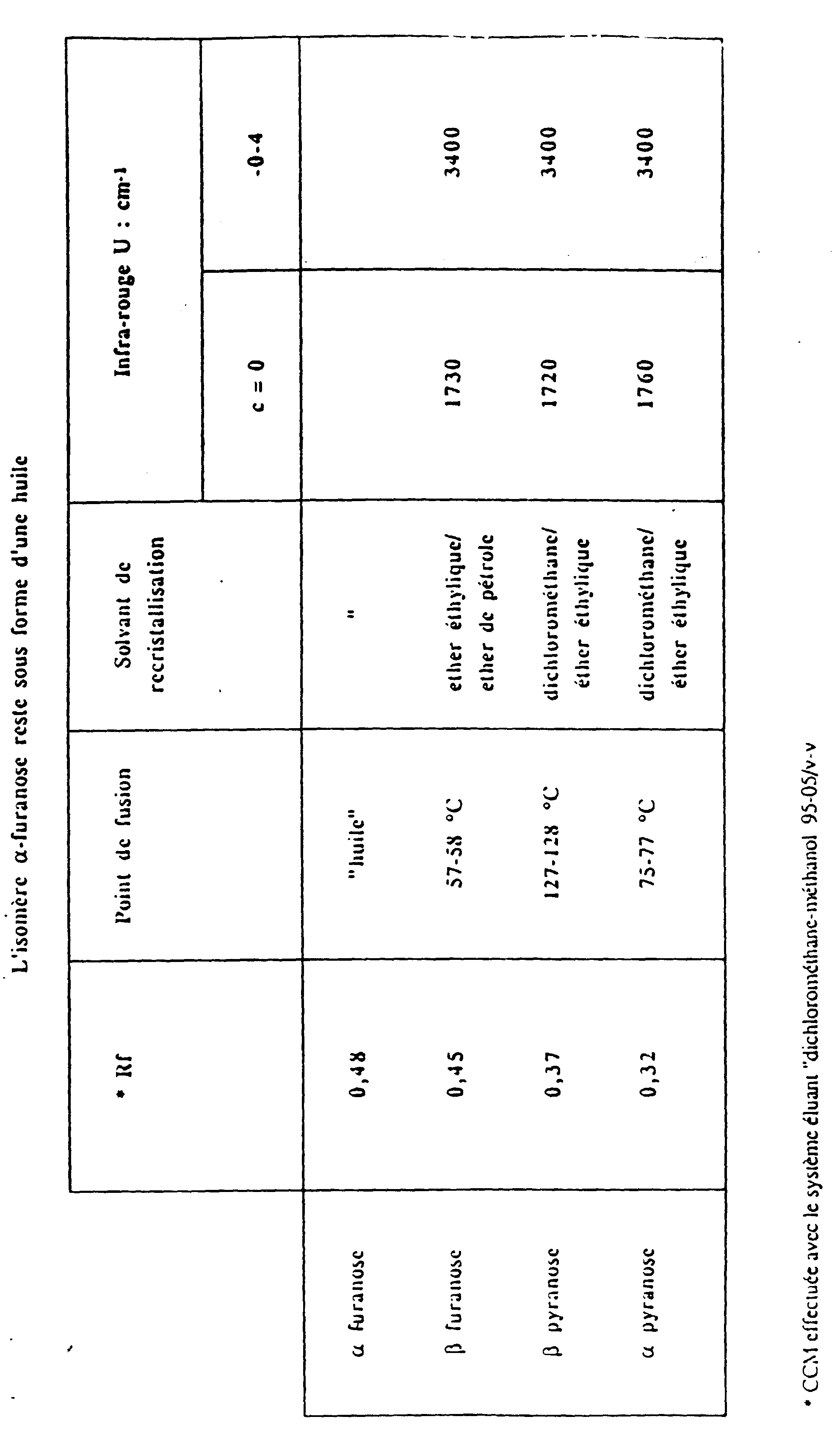

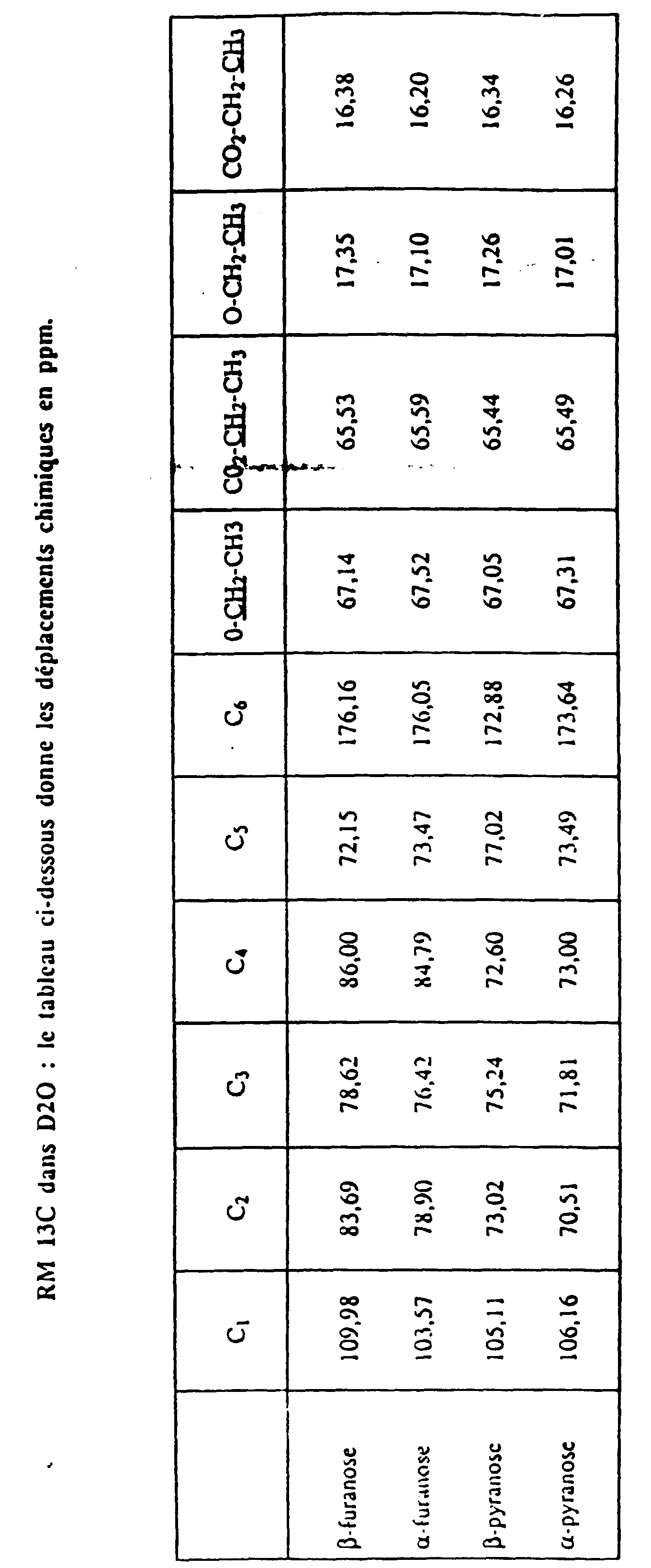

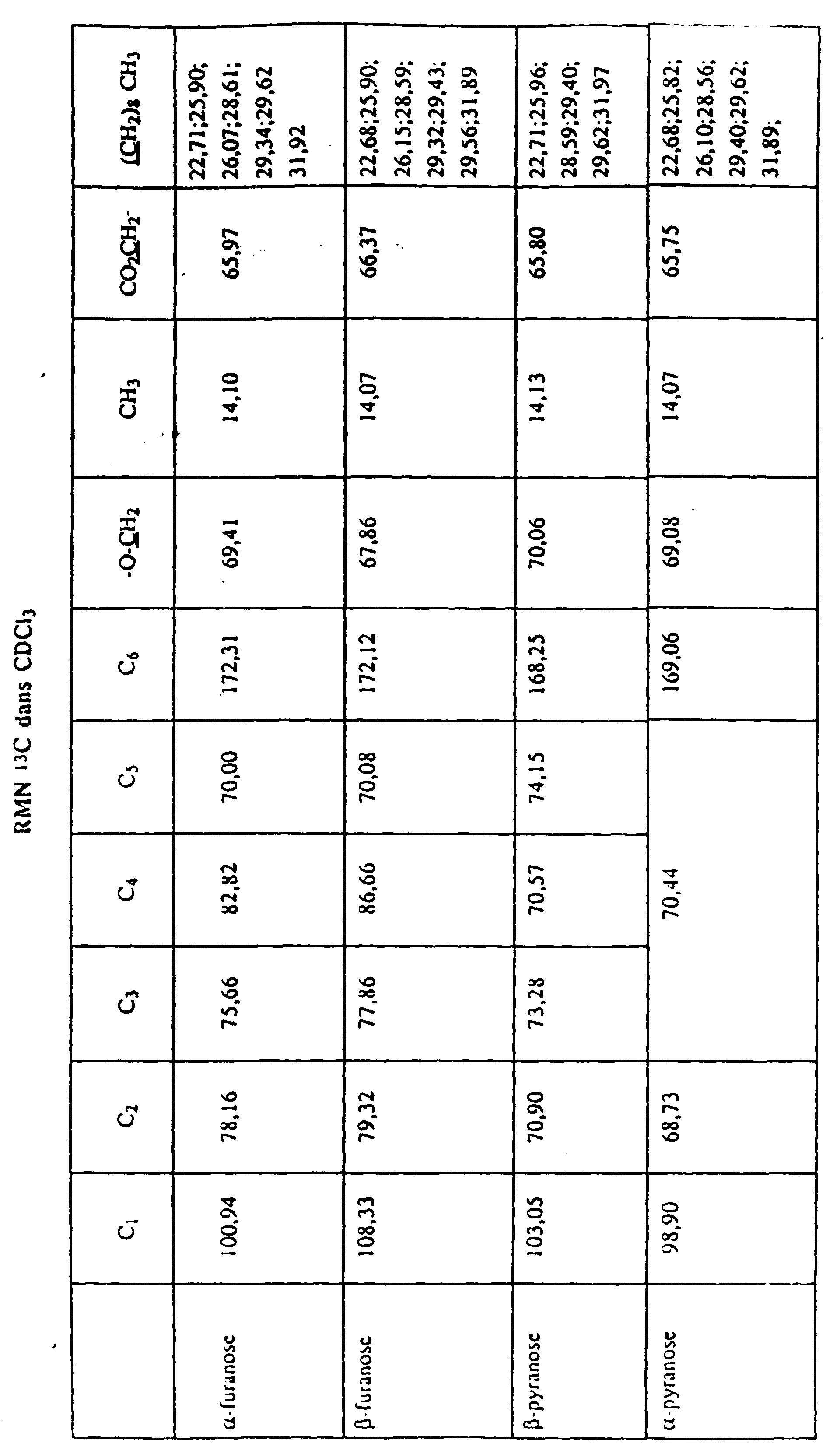

### 3) Synthèse n° 3

### Préparation du n-dodécyl D-galactoside uronate de n-dodécyle (I/R1 = R2 = dodécyle)

### Méthode A :

A 200 g (0,94 mole) d'acide D-galacturonique monohydraté, on ajoute 800 ml de diglyme, 702 g (3,77 moles) de n-dodécanol, et 7,36 g (0,08 mole) d'acide sulfurique concentré. On chauffe à 60 °C sous un vide de 18 mm de mercure pendant 24 heures. On amène à pH = 7 par de l'hydrogénocarbonate de sodium. On filtre les sels formés et on concentre le filtrat. Après chromatographie sur gel de silice, on récupère avec un rendement global de 70 % des isomères majoritaires n-dodécyl D-galactoside uronate de n-dodécyle (même système éluant que dans la synthèse n° 2).

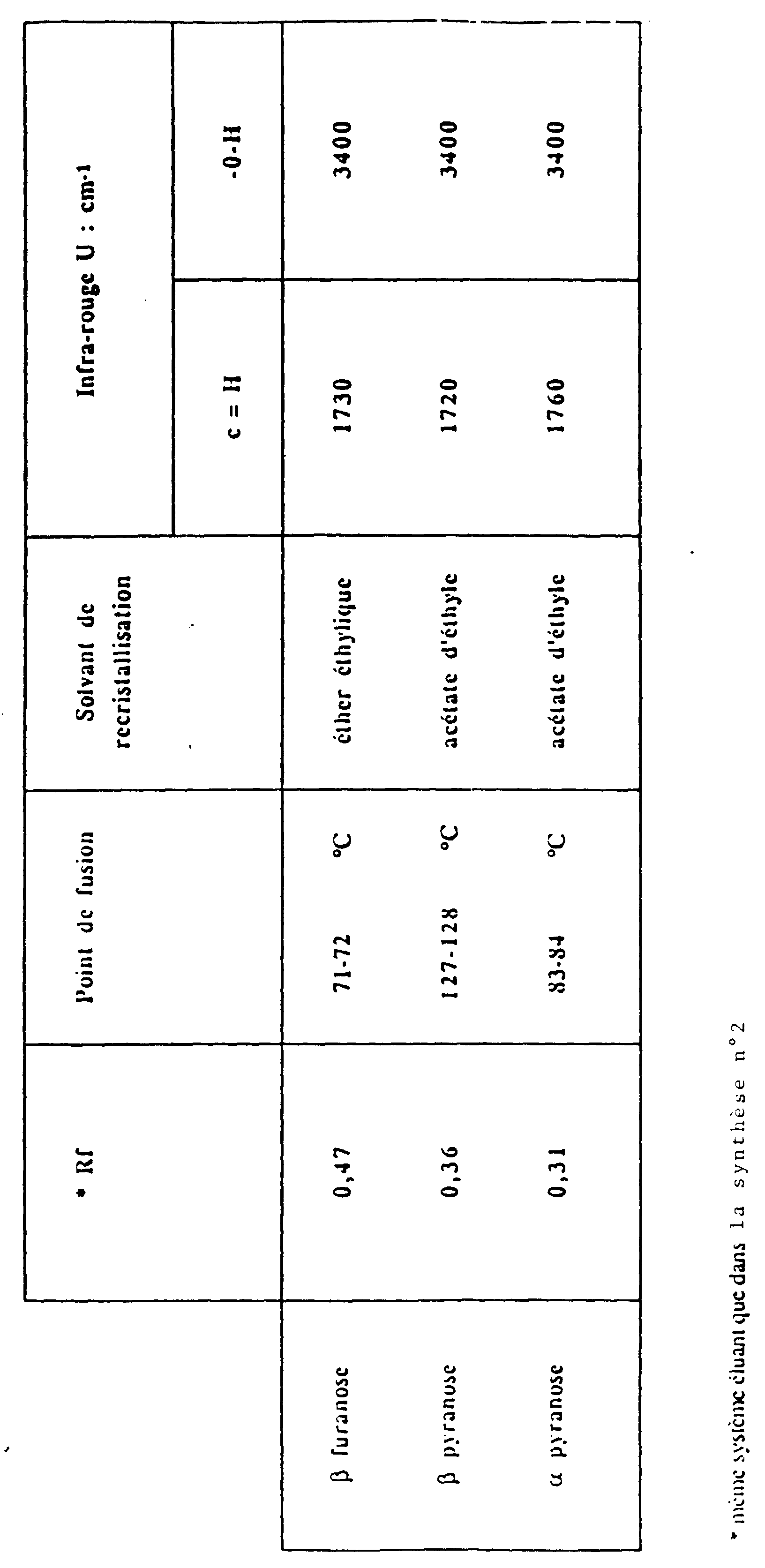

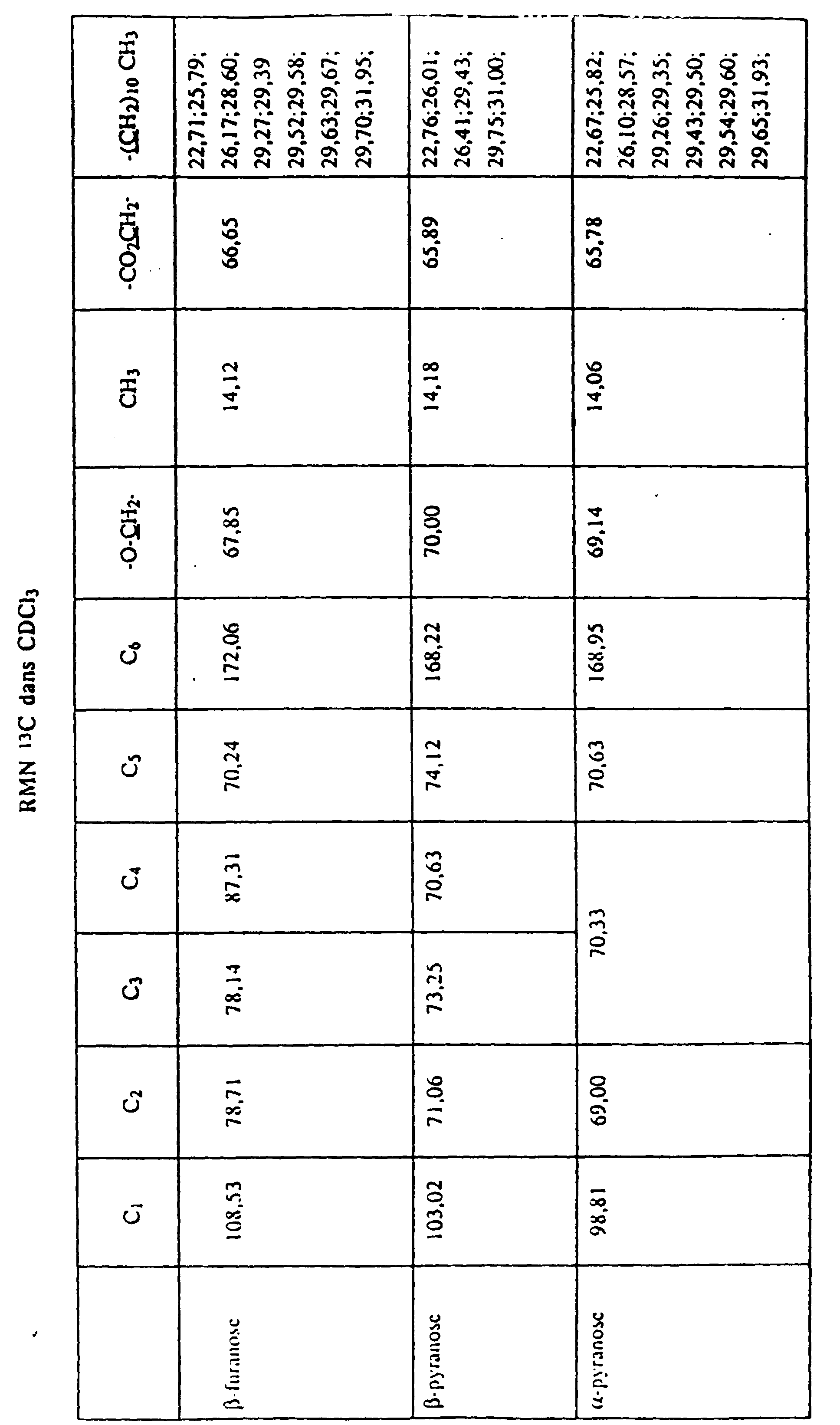

### Méthode B :

Effectuée avec les mêmes charges d'acide D-galacturonique et d'alcool gras que dans la méthode A, mais en utilisant comme catalyseur acide 4 g d'acide sulfosuccinique, et en conduisant la réaction à 70 °C sous un vide de 60 mm de mercure. Après 12 heures de réaction, le milieu réactionnel est chromatographié sur gel de silice (mêmes systèmes éluant que dans la synthèse n° 2). On récupère avec un rendement global de 80 %, les isomères majoritaires du dodécyl D-galactoside uronate de n-dodécyle décrits précédemment.

### 4) Synthèse n° 4

### Préparation du n-tetradécyl galactoside uronate de n-tetradécyle (I/R1 = R2 = n-tetradécyle)

Préparé selon la synthèse 3, méthode B, avec un rendement global 85%. Le catalyseur acide utilisé est l'acide paratoluène sulfonique monohydraté (5 g).
(voir tableaux pages 28 et 29)

### 5) Synthèse n° 5

### Préparation du n-hexadéyl galactoside uronate de n-hexadécyle (l/R1 = R2 = n-hexadécyle)

Préparé selon la synthèse 3, méthode B, avec un rendement global de 90 % mais le catalyseur acide utilisé est l'acide paratuolène sulfonique (6 g), et le système d'élution pour la purification chromatographique est le suivant dichlorométhane jusqu'à la sortie du n-hexadécanol en excès, puis le système dichlorométhane/méthanol (98/02-v/v). Les 4 isomères isolés cristallisent après évaporation des solvants d'élution et sont ensuite recristallisés. (Voir tableaux pages 30 et 31)

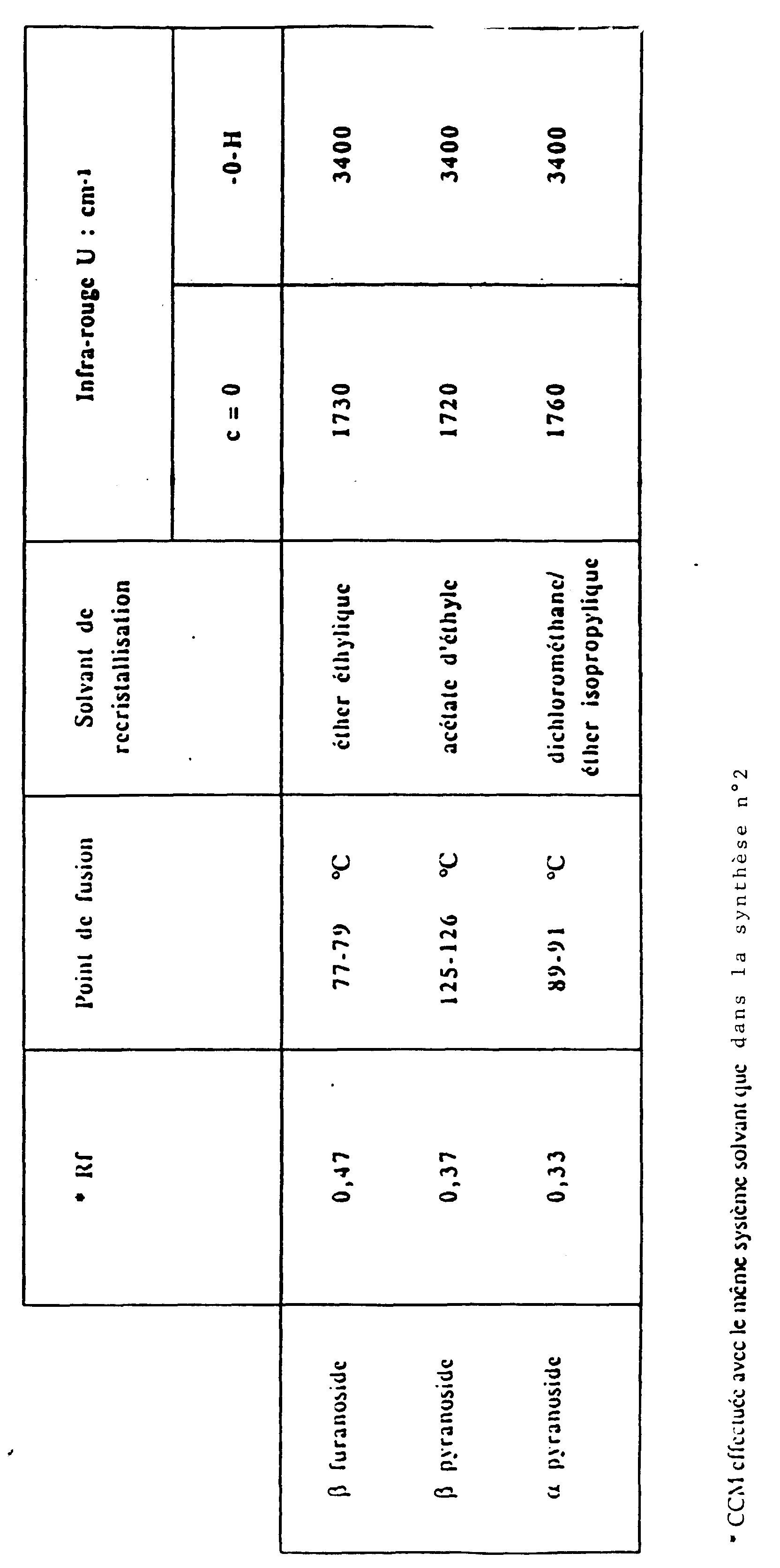

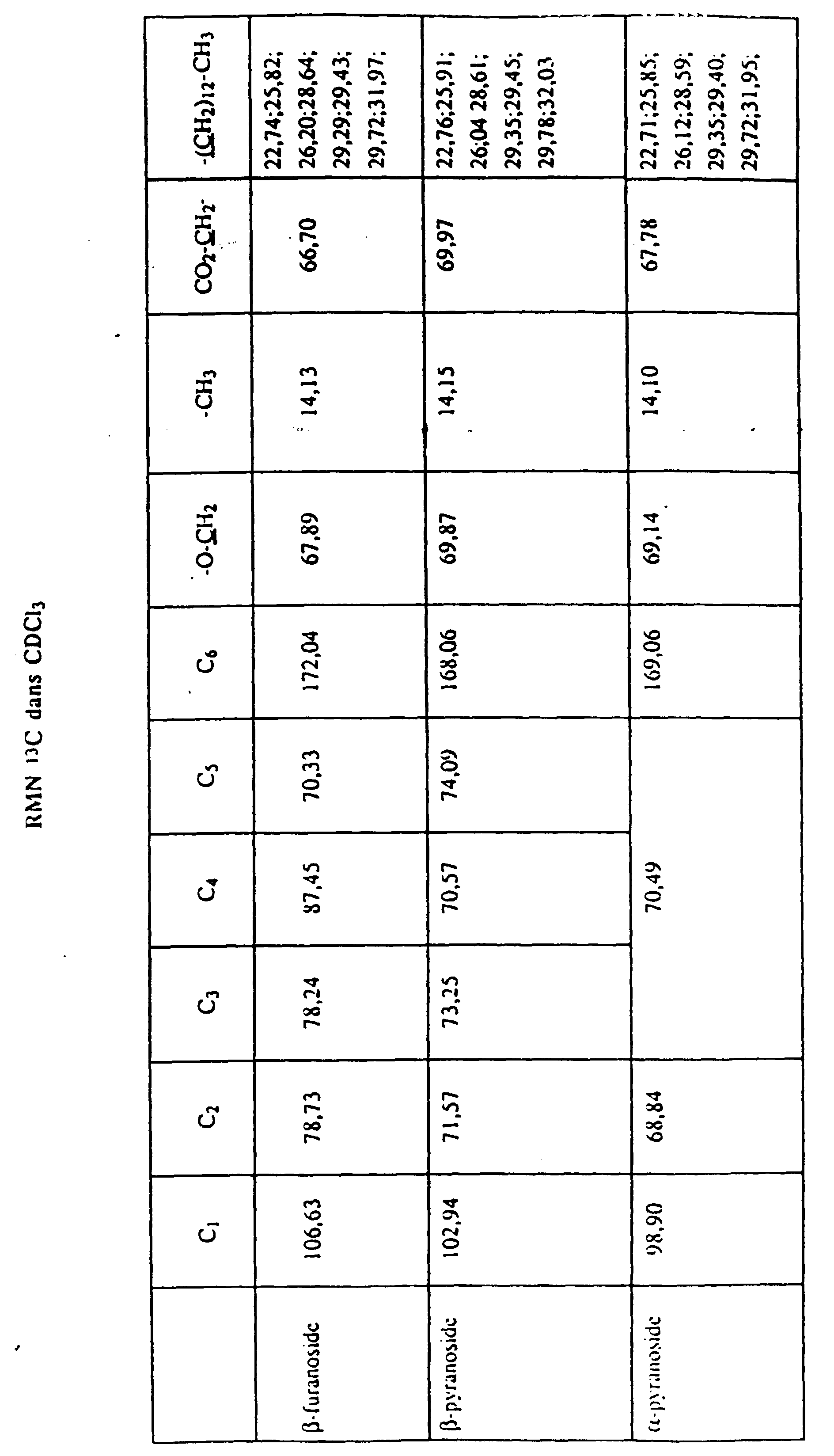

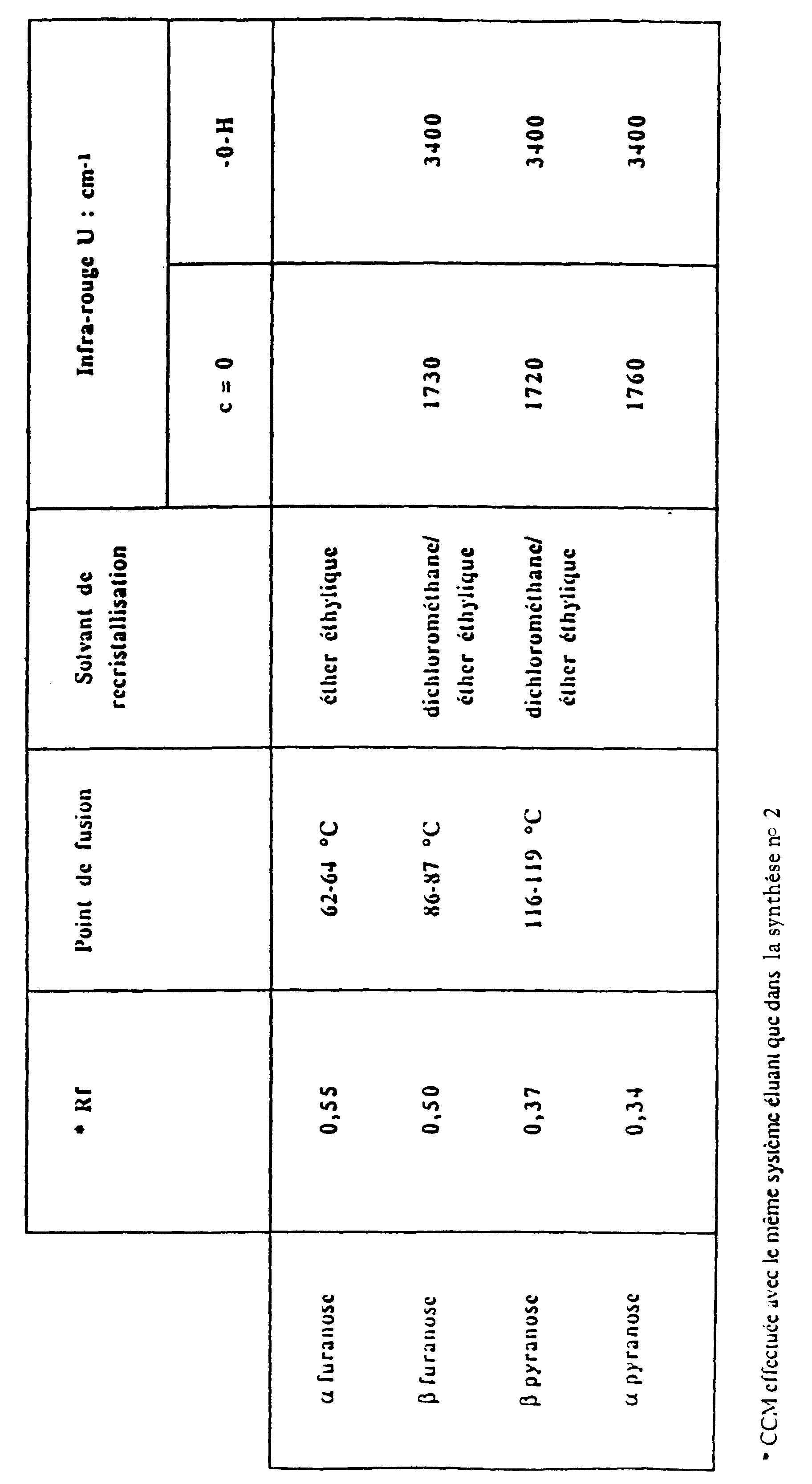

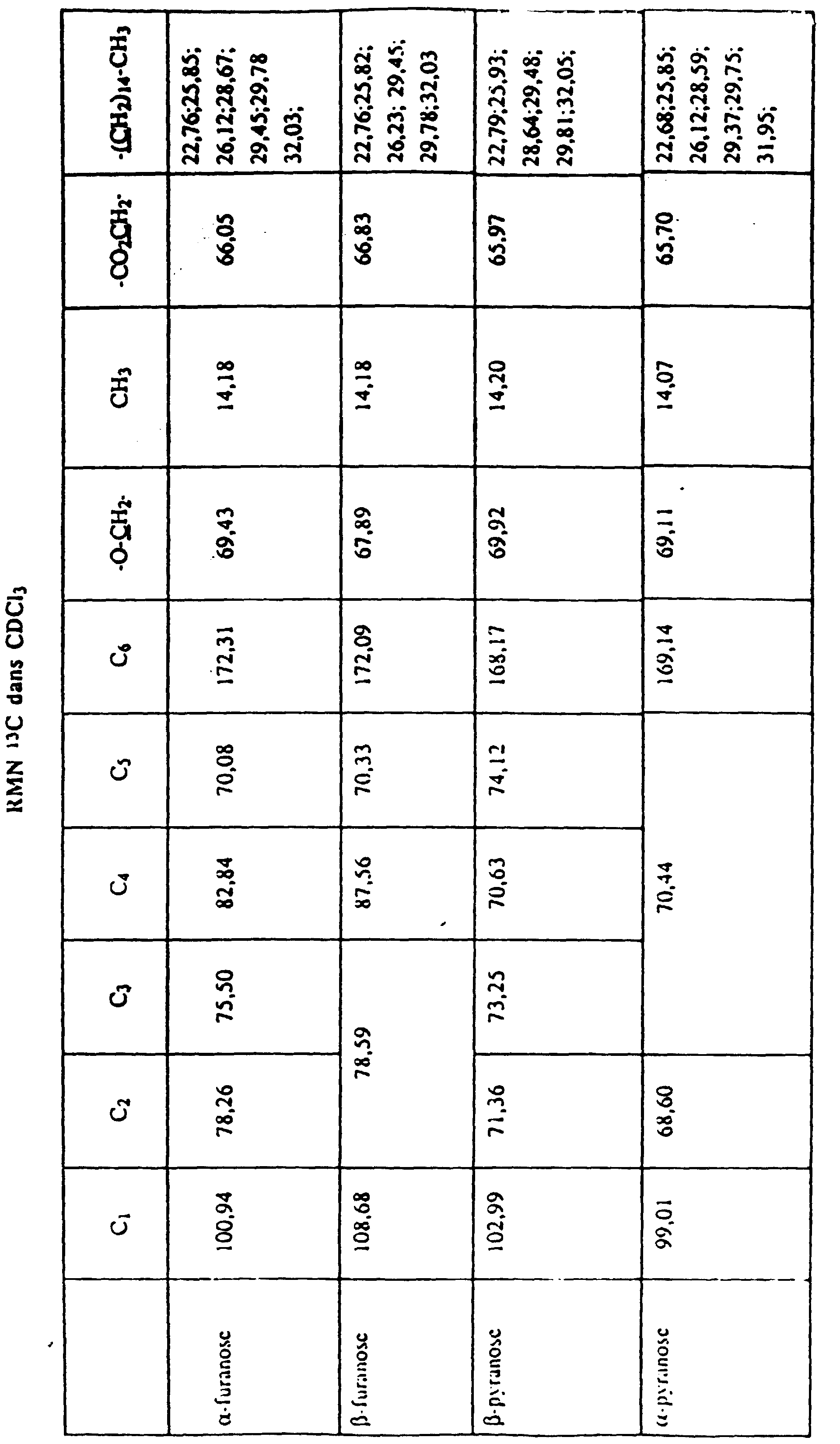

### 6) Synthèse n° 6

### Préparation du n-octadécyl D-galactoside uronate de n-octadécyle (I/R1 = R2 =n-octadécyle)

### Méthode A:

A 200 g (0,94 mole) d'acide D-galacturonique monohydraté, on ajoute 508,5 g (1,88 mole) de n-octadécanol, 800 ml de diglyme et 10,2 g (5,53 ml ; 0,10 mole) d'acide sulfurique concentré, On porte à 60 °C sous un vide de 18 mm de Hg, pendant 10 heures. On ajoute alors de l'hydrogénocarbonate de sodium dans le milieu réactionnel jusqu'à l'obtention d'un pH de 7. On laisse refroidir; le milieu réactionnel qui se solidifie est additionné de 500 ml de tétrahydrofuranne, agité, filtré sur frîné n° 3. Du filtrat concentré précipite le n-octadécyl-D-galactoside uronate de n-octadécyle avec un rendement global de 94 %. Une chromatographie analogue à celle effectuée dans la synthèse 5 permet l'isolement des trois isomères majoritaires qui précipite lors de l'évaporation des solvants d'élution et qui sont ensuite recristallisés.

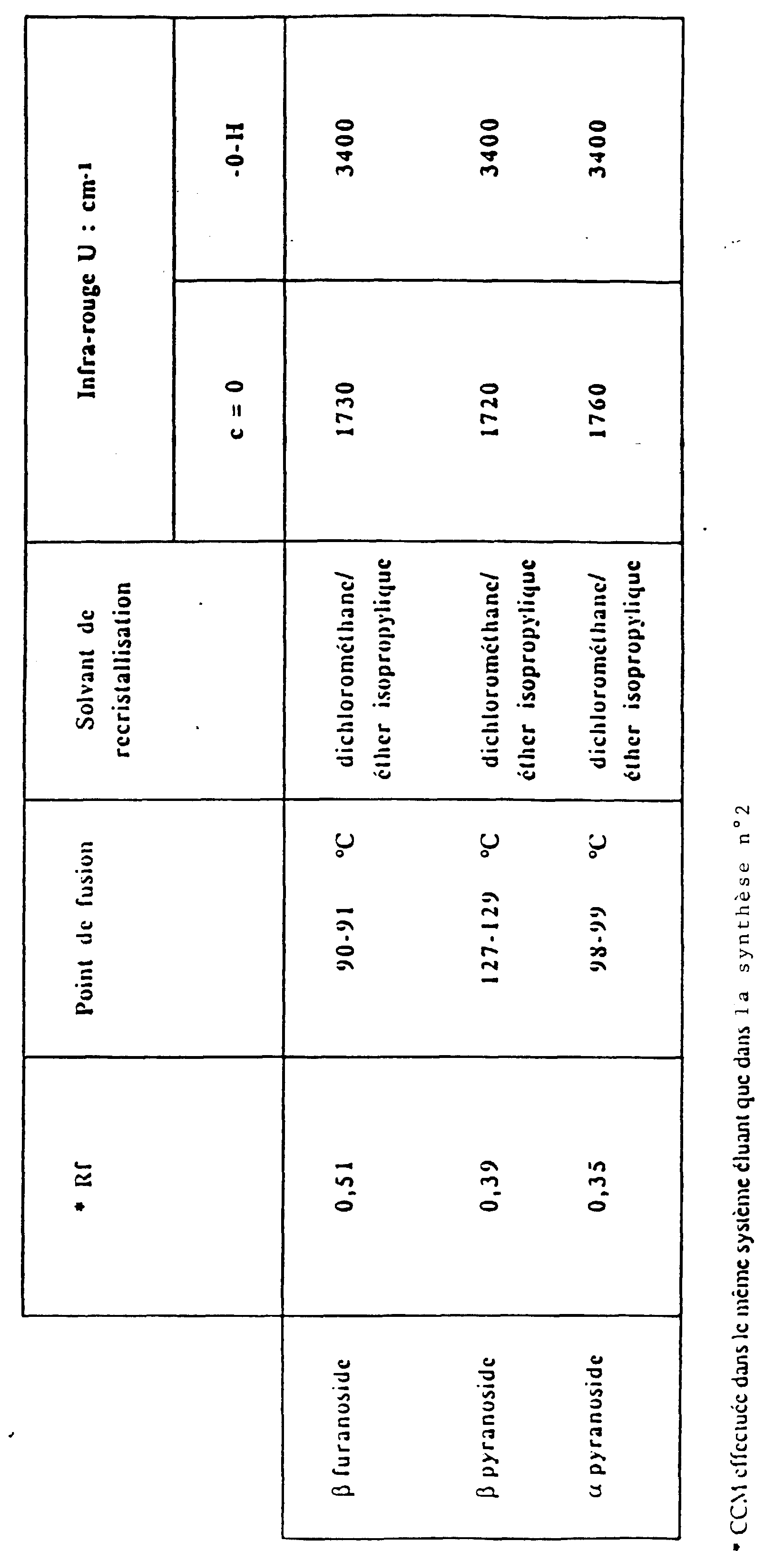

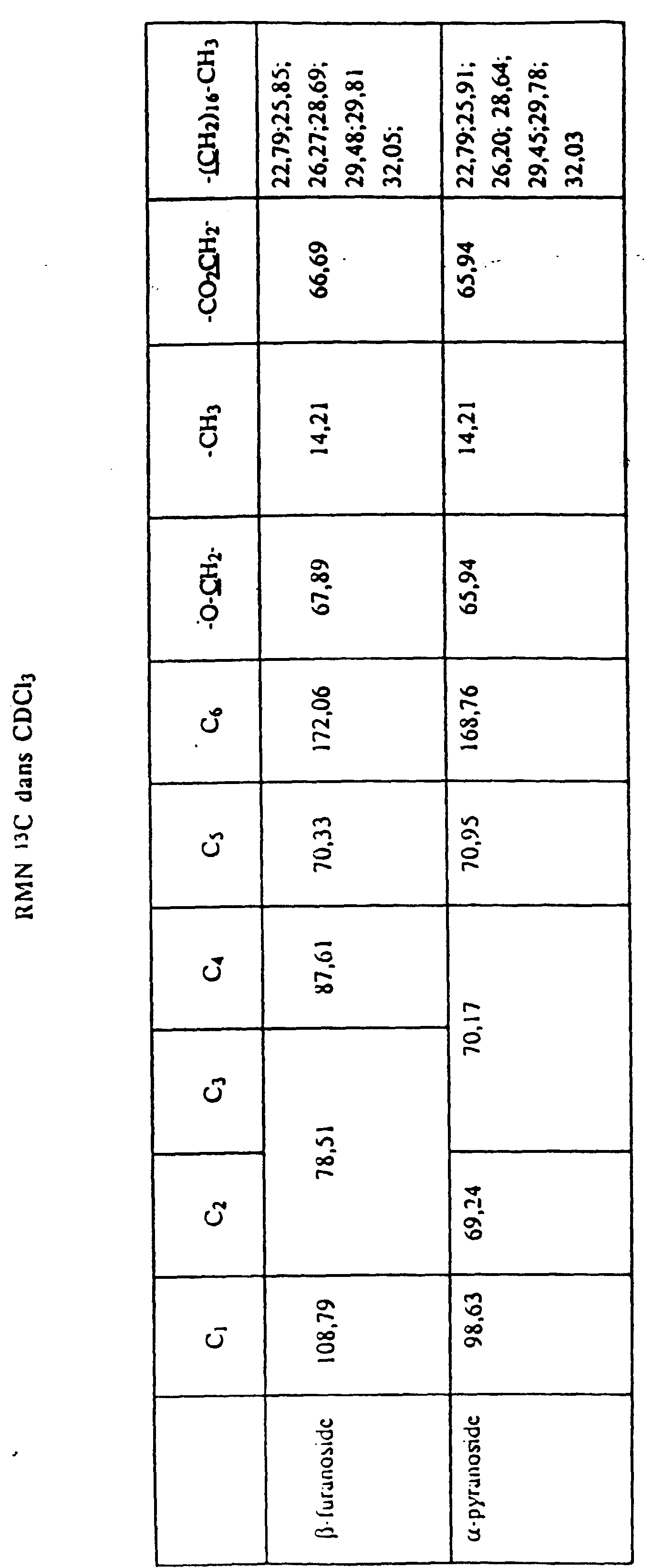

### 7) Exemple n° 7

### Préparation de l'octyl D-galactoside uronate de sodium (I/R1 = octyle et R2 = sodium)

### Méthode A:

A une solution de 100 g (0,24 mole) du mélange d'octyl D-galactoside uronate d'octyle préparé selon la synthése 1 dans 200 ml de tétrahydrofuranne est additionnée une solution de 9,4 g (0,24 mole) d'hydroxyde de sodium dans 50 ml d'eau. Après 1 heure d'agitation à 30°C, le mélange d'octyl D-galactoside uronate de sodium qui décante est repris dans 200 ml d'éthanol absolu. Le mélange est agité pendant 30 minutes. Des cristaux qui se forment sont filtrés, lavés avec 200 ml d'éthanol absolu, puis séchés. Ils correspondent au mélange d'octyl D-galactoside uronates de sodium ainsi obtenu de façon quantitative.

Partant de chacun des 4 octyl D-galactoside uronates d'octyle purifiés selon la synthèse 1, on obtient, selon ce même protocole, et de façon quantitative, chacun des 4 octyl D-galactoside uronates de sodium isomères purs dont les caractéristiques physico-chimiques sont résumées ci-après.

RMN ¹³C DANS D₂O :
le tableau ci-dessous donne les déplacements chimiques en ppm

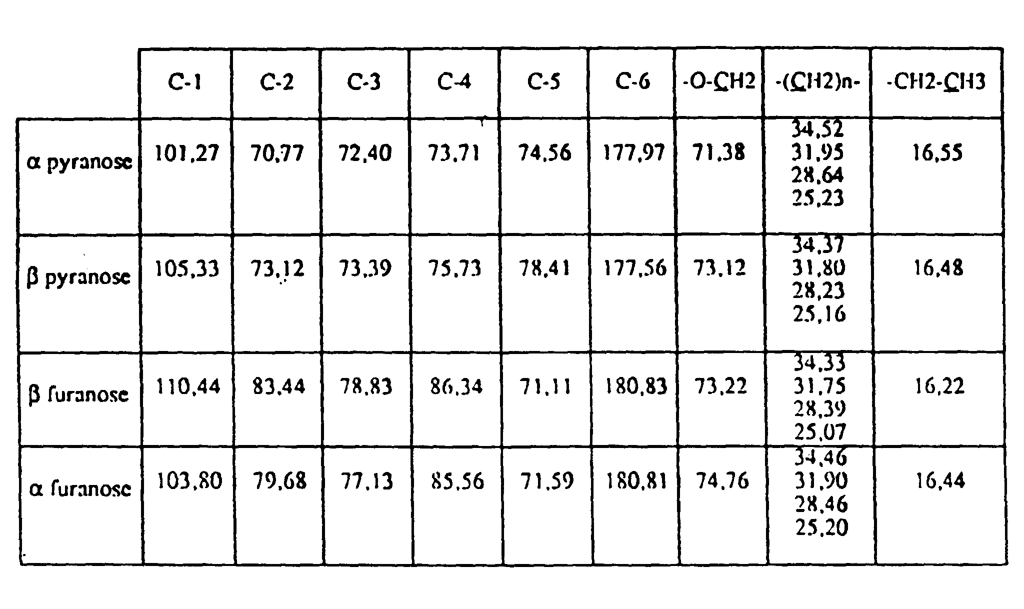

### Méthode B:

A 40 g (0,096 mole) d'un mélange d'octyl D-galactoside uronates d'octyle en solution dans 200 ml de l'éther diméthylique de l'éthylène glycol, on ajoute 3,84 g (0,096 mole) d'hydroxyde de sodium en solution dans 20 ml d'eau et 0,2 ml d'hydroxyde de tétrapropylammonium. Après 30 minutes d'agitation à 20 °C, on évapore le solvant sous pression réduite et le résidu est repris avec 200 ml d'acétate d'éthyle. Le précipité formé est filtré, lavé à l'acétate d'éthyle (50 ml) puis séché. On obtient quantitativement le mélange d'octyl D-galactoside uronatee de sodium correspondant.

### 8) Exemple n° 8

### Préparation du dodécyl β-D-galactofuranoside uronate de sodium (I/R1 : dodécyle et R2 = sodium)

A une solution de 100 g (0,19 mole) de dodécyl β-D-galactofuranoside uronate de dodécyl dans 500 ml d'éthanol absolu est ajoutée une solution de 7,6 g (0,19 mole) d'hydroxyde de sodium dans 20 ml d'eau. Après 1 H 30 minutes d'agitation à 30 °C, filtration, lavage du précipitation avec de l'éthanol absolu (50 ml x 4) à 40 °C, séchage, on obtient avec un rendement toujours supérieur à 95 % le dodécyl α-D-galactofuranoside uronate de sodium, dont les caractéristiques physico-chimiques sont données ci-après :
RM ¹³C dans D₂O : les déplacements chimiques sont exprimés en ppm

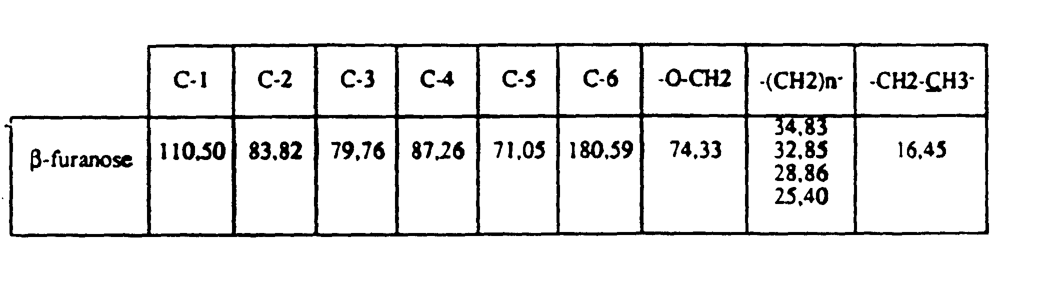

### 9) Exemple n° 9

### Préparation de l'acide octyl (α-D-galactopyranoside) uronique

50 g (0.15 mole) d'octyl (α-D-galactopyranoside) uronate de sodium préparé selon l'exemple 7 sont solubilisés dans l'eau (solution à 30 %). Après passage sur 400 ml d'une résine cationique de type IR 120 H⁺, concentration sous vide, on récupère quantitativement l'acide (α-D-galactopyranoside) uronique sous forme d'une gomme dont les caractéristiques physico-chimiques sont indiquées ci-après:

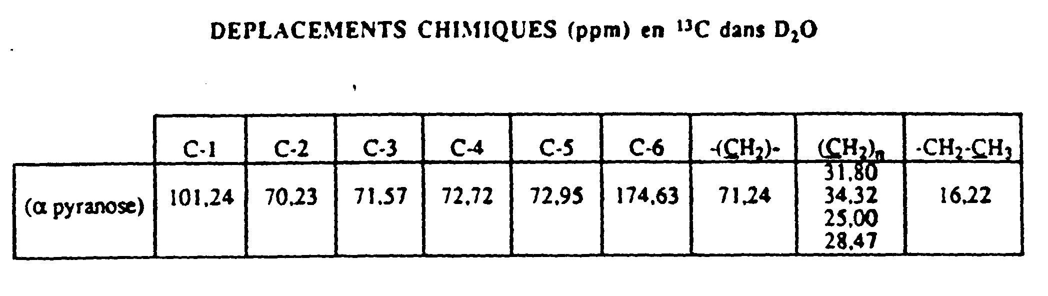

### 10) Exemple n° 10

### Préparation de l'acide n-décyl (β-D-galactofuranoside) uronique

A 240 mg (0,51 mmoles) de n-décyl(β-D-galactofuranoside) uronate de n-décyle solubilisés dans 5 ml de THF, on ajoute 20 ml de soude 2,5 N a on laisse évoluer à temperature ambiante pendant 3 heures. On ajoute 30 ml d'éther éthylique, puis après décantation la phase aqueuse est lavée avec 30 ml d'éther éthylique. La phase aqueuse est acidifiée pu HCl 50 %, à 0 °C, jusqu'à pH = 2, puis extraite trois fois avec 30 ml d'acétate d'éthyle. Les phases organiques sont rassemblées, lavées avec 30 ml d'eau, séchées sur sulfate de magnésium (2 g) et évaporées. On obtient alors 140 mg d'acide n-décyl-(β-D-galactofuranoside) uronique (82 % de rendement) qui se présente sous la forme d'une gomme.
* Rf =0,67 (dichlorométhane/méthanol - I/1 - v/v)
* RMN ¹³C dans CD₃OD

| C1 | C2 | C3 | C4 | C5 | C6 | -CH₂-O- | -CH₃ | -(CH₂)₈-CH₃⁻ |
|---|---|---|---|---|---|---|---|---|
| 110,12 | 83,96 | 78,76 | 85,66 | 71,09 | 167,16 | 69,76 | 15,21 | 24,42;27,97; 31,25;31,38; 31,46;33,77; |

### 11) Exemple n° 11

### Préparation de l'acide n-décyl (α-D-galactopyranoside) uronique

Le protocole effectué sur 360 mg (0,76 mmole de substrat est analogue à celui décrit dans l'exemple n° 10, mais en utilisant à la place de l'acétate d'éthyle un mélange acétate d'éthyle/n butanol (1/1-v/v), lors des dernières extractions.

On obtient 230 mg (90 % de rendement) de l'acide n décyl (α-D-galactopyranoside) uronique, qui se présente sous la forme de cristaux blancs et dont les caractéristiques physico-chimiques sont indiqués ci-après:
Rf: 0,22 (dichlorométhane/méthanol - 1/1-v/v)
RMN ¹³C dans CD₃OD

| C1 | C2 | C3 | C4 | C5 | C6 | -CH₂-O- | -CH₃ | -(CH₂)₈-CH₃- |
|---|---|---|---|---|---|---|---|---|
| 101,24 | 70,28 | 71,69 | 72,45 | | 173,21 | 70,58 | 15,21 | 24,37;27,91; 31,11;31,22; 31,39;33,71; |

### 12) Exemple n° 12

### Préparation de l'acide n-décyl (β-D-galactopyranoside) uronique :

La préparation, à partir de 140 mg (0.29 mmoles) de n-décyl-β-D-galactopyranoside uronate de n-décyl, et selon le protocole opératoire décrit dans l'exemple n° 11, de l'acide n-décyl(β-D-galactopyranoside) uronique, se fait avec un rendement de 85 % (82 mg) ; il se présente sous la forme d'une poudre blanche dont le résumé des caractéristiques physico-chimiques sont indiquées ci-après :
Rf: 0,38 (dichlorométhane/méthanol - 1/1-v/v)
RMN ¹³C dans CD₃OD

| C1 | C2 | C3 | C4 | C5 | C6 | -(CH₂₎-O- | -CH₃ | -(CH₂)₈-CH₃- |
|---|---|---|---|---|---|---|---|---|
| 105,33 | 72,72 | 75,26 | 72,12 | 76,07 | 172,75 | 71,90 | 15,18 | 24,45;27,81; 31,17;31,36; 31,41;31,46; 33,77; |

### EXEMPLE N° 13 :

Pouvoir moussant d'alkyl galactoside uronates de sodium selon l'invention (norme NFT 73404).

Composés I / R1 = octyl, décyl, dodécyl, tetradécyl, hexadécyl.
R2 est le sodium.

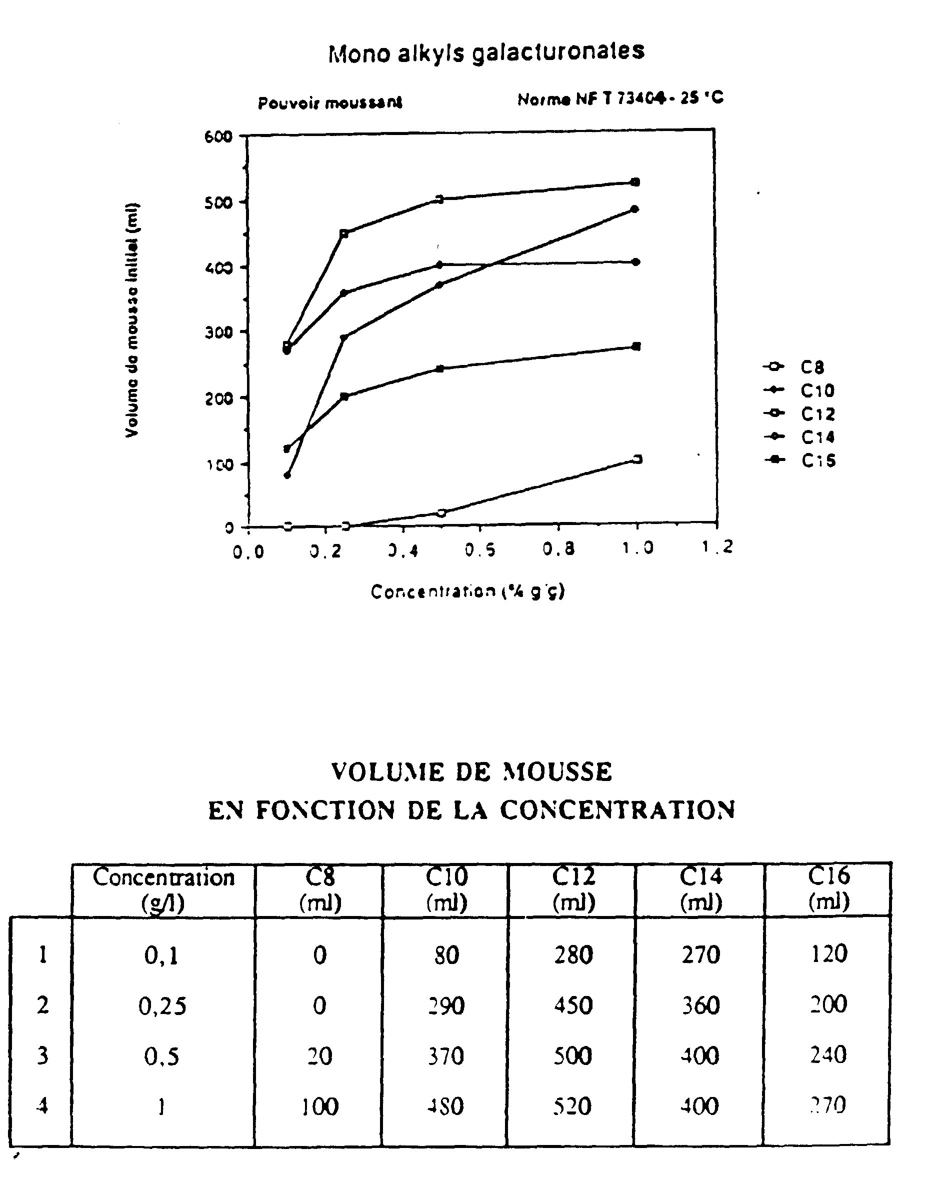

### EXEMPLE COMPARATIF N° 14 :

Comparaison du pouvoir moussant de différents tensio-actifs connus et du dodécyl galactoside uronate de sodium - I / R1 = dodécyle et R2 est le sodium (norme NFT 73404).

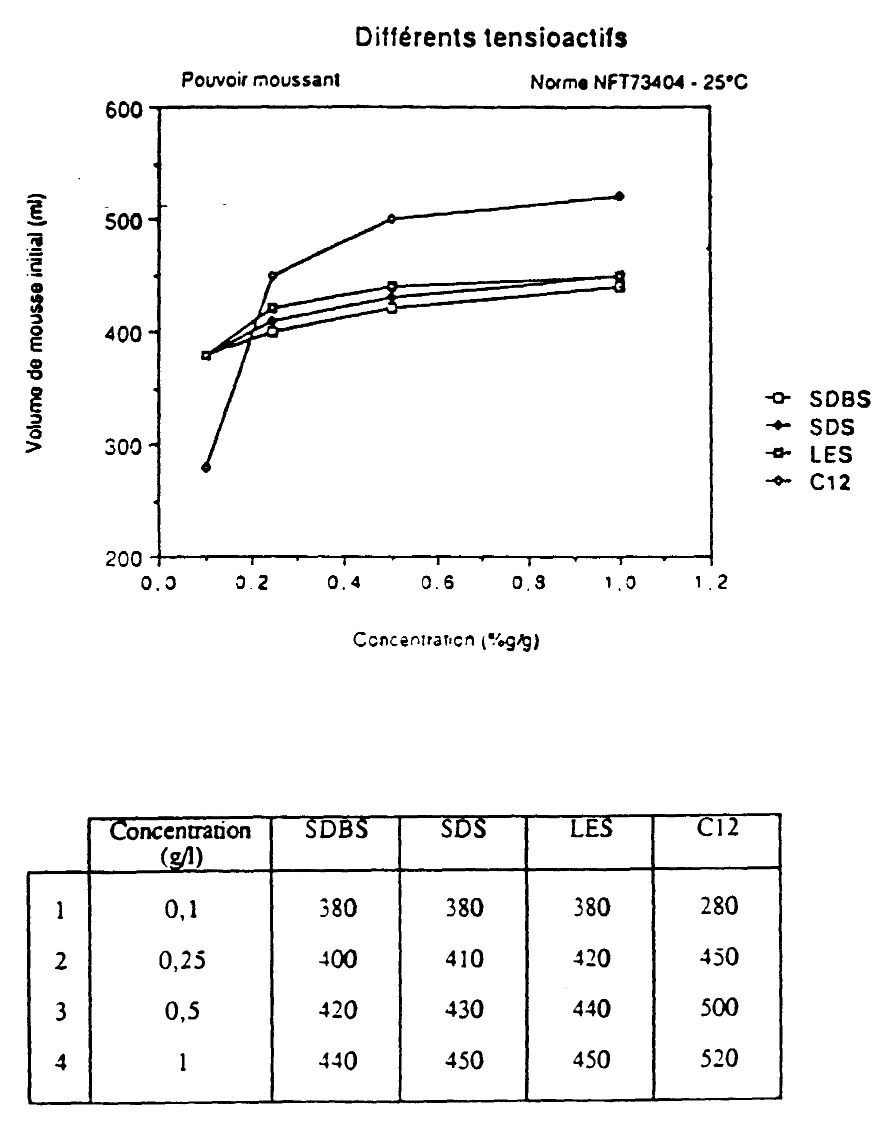

Les concentrations sont exprimées en g/l. Les volumes de mousse en ml.

### EXEMPLE COMPARATIF N° 15 :

Comparaison du pouvoir moussant d'une solution à 1 % d'alkyl galactoside uronate de sodium selon l'invention (I / R1 = octyl, décyl, dodecyl, tétradécyl, hexadecyl et R2 est le sodium) et des produits de référence (norme NFT 7340 - 25°C - volume initial de mousse).

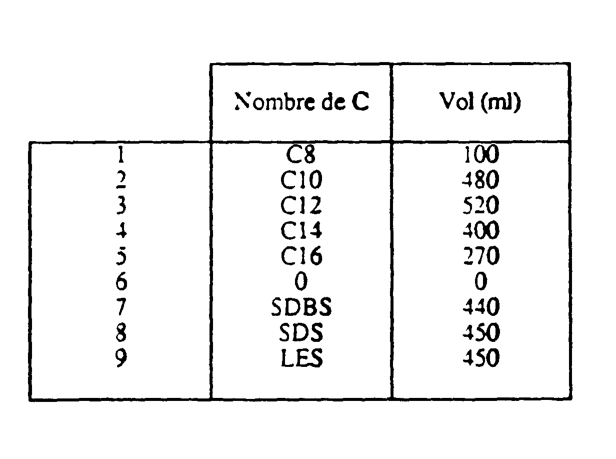

### EXEMPLE N° 16 :

Etude de la stabilité de la mousse des alkyl galactoside uronates de sodium selon l'invention (I / R1 = octyl, décyl, dodécyl, tetradécyl, hexadécyl, et R2 est le sodium).
Norme NFT 73404.

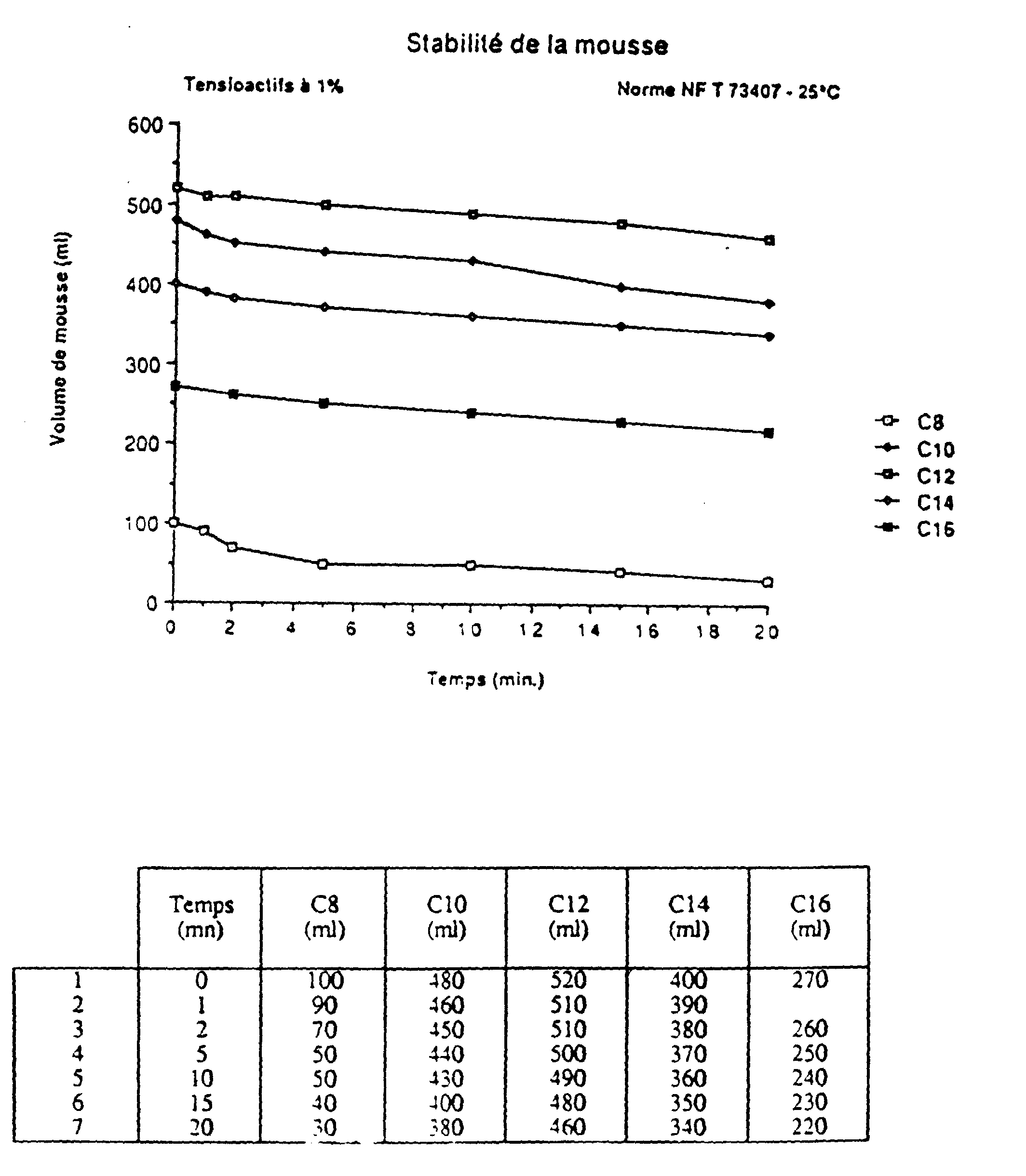

### EXEMPLE COMPARATIF N° 17 :

Etude comparative de la stabilité de la mousse d'alkyl galactoside uronates de sodium selon l'invention (I / R1 = decyl, docécyl, tetradécyl, et R2 est le sodium) et de tensio-actifs connus.
Norme NFT 73404.

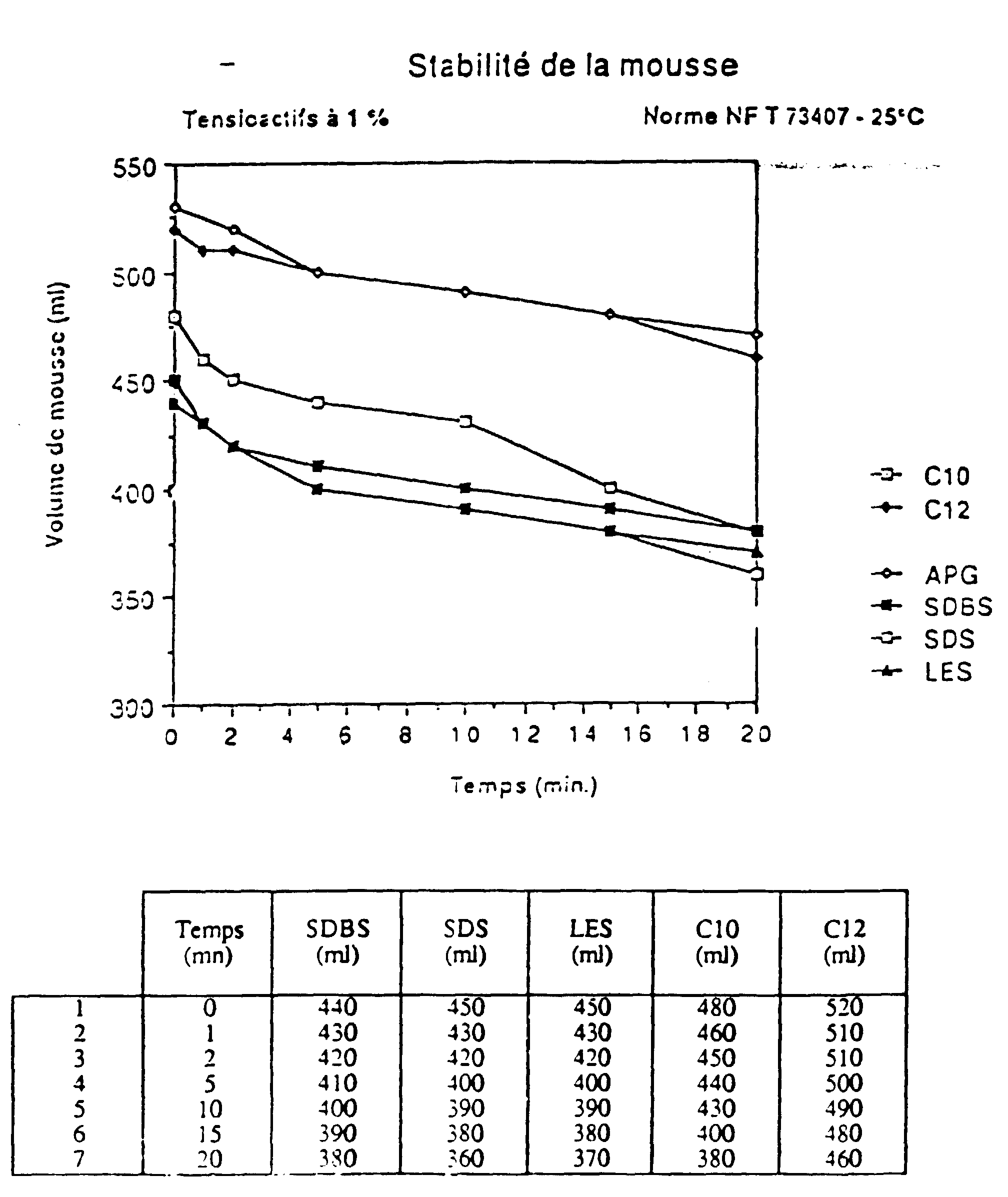

### EXEMPLE N° 18 :

Mesure de l'absorption des solutions à 1 % d'alkyl galactoside uronates de sodium selon l'invention.
Composés I/ R1 = octyl, décyl, dodécyl, tetradécyl, hexadécyl et R2 est le sodium.

Tensiomètre KRUSS - 25°C.

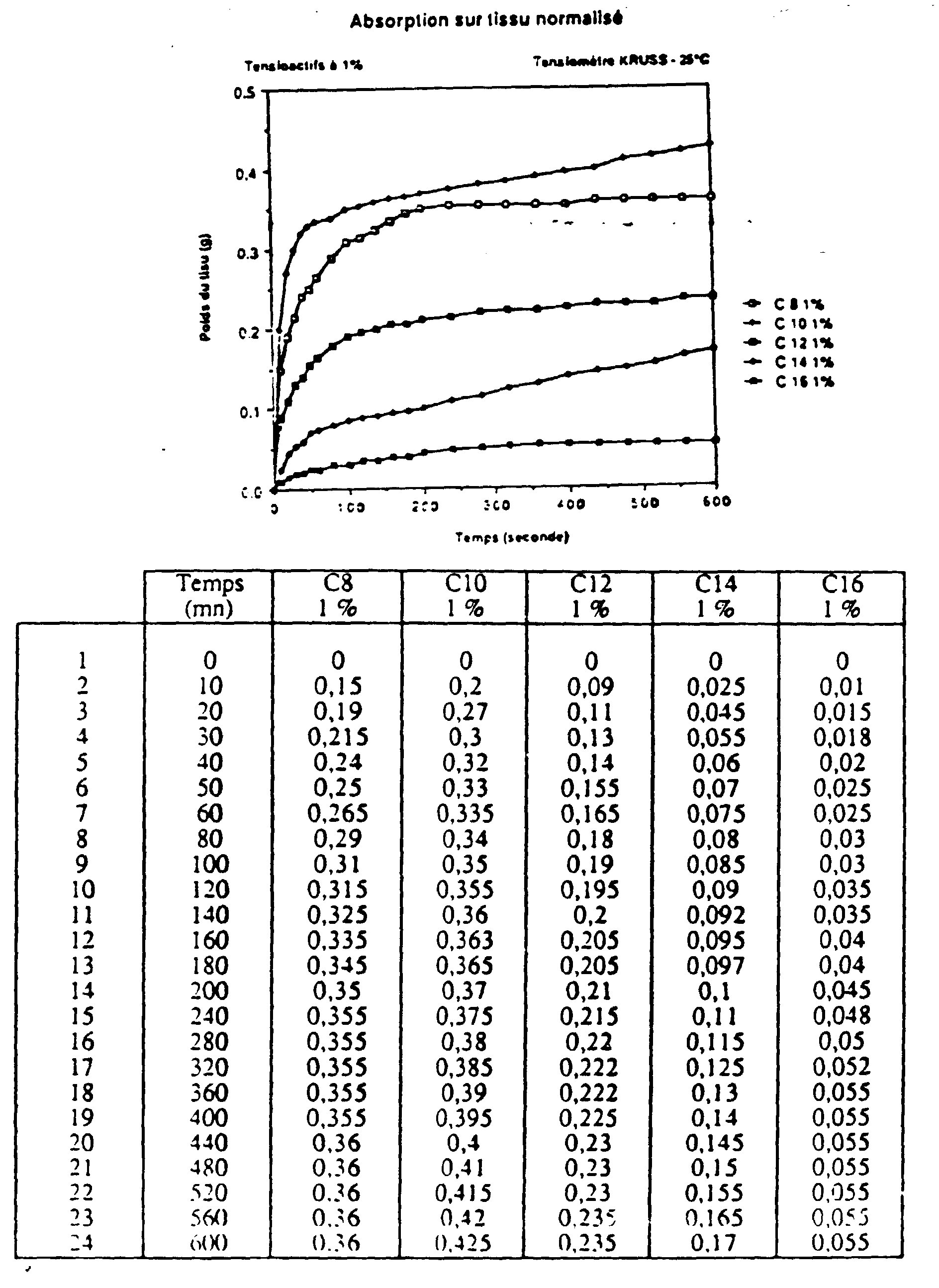

### EXEMPLE COMPARATIF N° 19 :

Mesure comparative de l'absorption de solutions à 1 % de tensio-actifs connus et du décyl galactoside uronate de décyl (composé I) R1 = décyl et R2 est le sodium.

Tensiomètre KRUSS - 25°C.

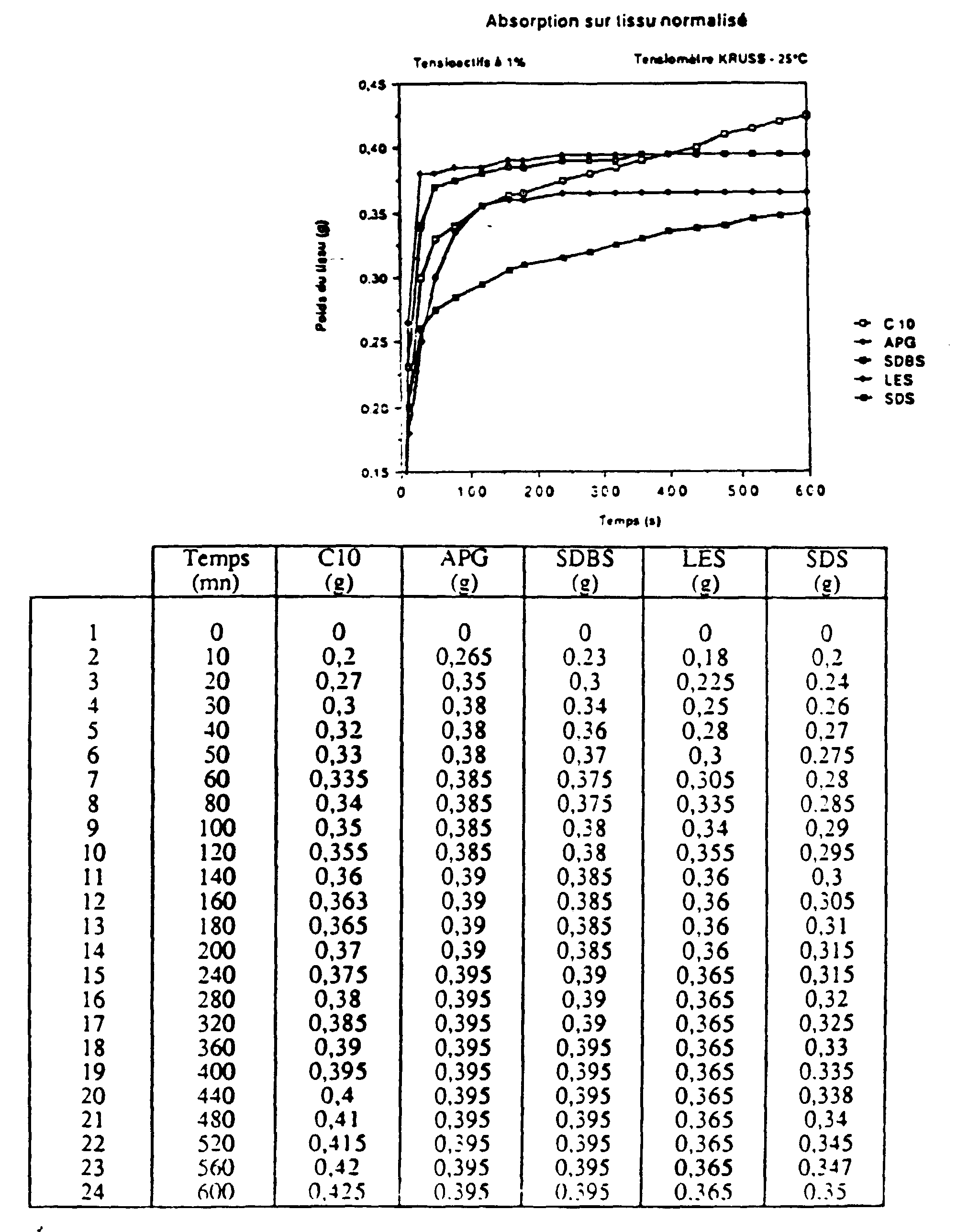

### EXEMPLE N° 20 :

Mesures de l'angle de mouillage, sur du polyéthylène, de solutions à 1 % d'alkyl galactoside uronates selon l'invention (composé I).
R 1 = décyl, dodécyl, tetradécyl, hexadécyl, octadécyl et R2 est le sodium.

Tensiomètre KRUSS - 25°C.

### EXEMPLE COMPARATIF N° 21 :

Mesures comparatives de l'angle de mouillage de solution à 1 % de décyl galactoside uronate de sodium (composé I / R1 = décyl et R2 est le sodium) et de tensio-actifs connus.

| Tensio-actifs (1 %) | Angle de contact(°) |
|---|---|
| GA C10 | 4 |
| GAC12 | 20 |
| GAC14 | 55 |
| GAC16 | 60 |
| GAC18 | 68 |
| SDBS | 22 |
| SDS | 60 |
| LES | 62 |
| | |

### EXEMPLE N° 22 :

Mesure de tensions superficielles de solutions à 1 % d'alkyl galactoside uronate de sodium selon l'invention (composé I / R1 = octyl, décyl, dodécyl, tetradécyl, hexadécyl, octadécyl et R2 est le sodium).

### EXEMPLE COMPARATIF N° 23 :

Mesures comparatives des tensions superficielles d'une solution à 1 % d'octyl galactoside uronate de sodium (I / R1 = octyl et R2 est le sodium) et de solutions de tensio-actifs connus.

| Tensio-actifs (1 %) | γ(mN/m) |
|---|---|
| GA C8 | 23 |
| GAC10 | 23 |
| GAC12 | 30 |
| GAC14 | 33 |
| GAC16 | 40 |
| GAC18 | 44 |
| APG | 26 |
| SDBS | 30 |
| SDS | 35 |
| LES | 35 |

### EXEMPLE N° 24 :

Concentrations micellaires critiques des alkyls galactosides uronates de sodium selon l'invention (composé I / R1 = octyl, décyl, dodécyl, tetradécyl, hexadécyl, octadécyl et R2 est le sodium).

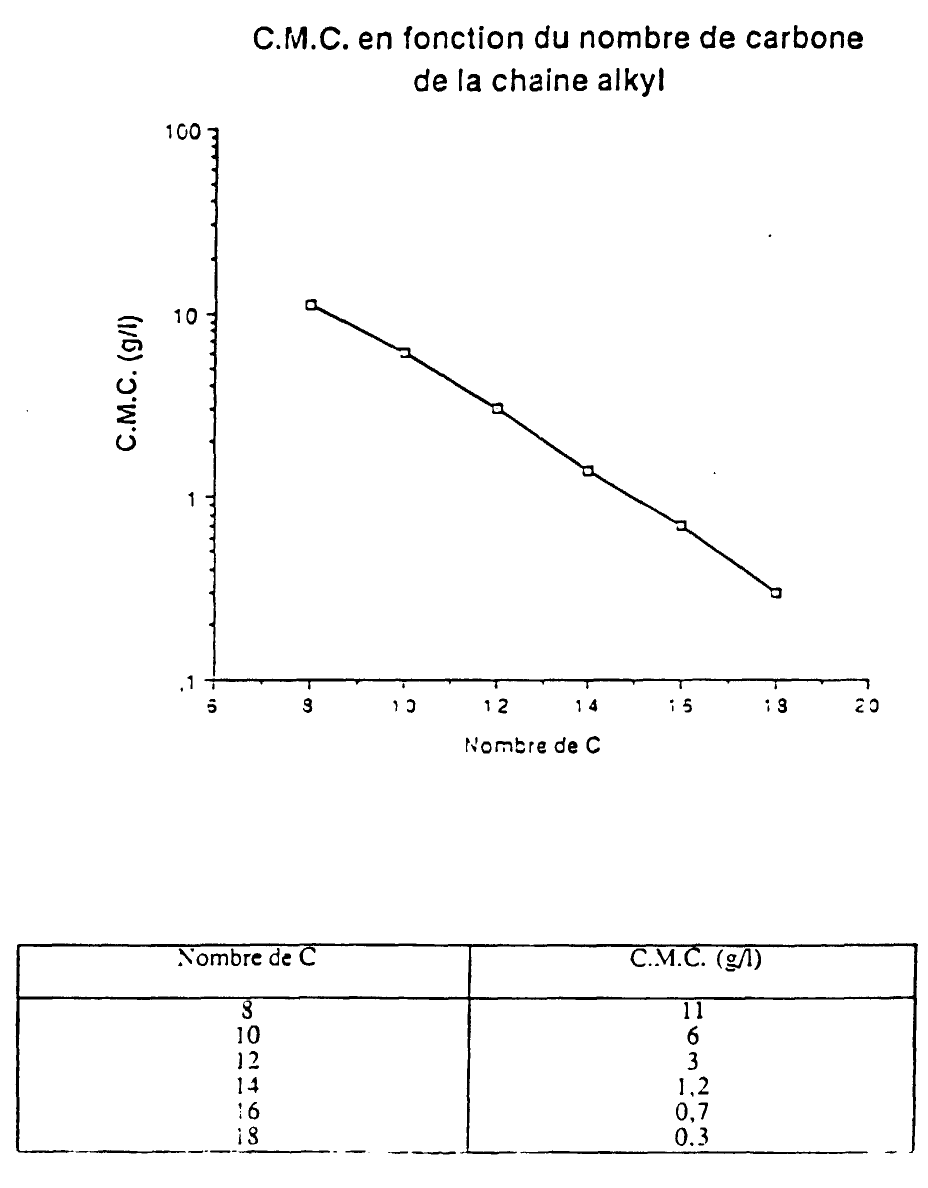

**EXEMPLES N° 25 A 27 :**

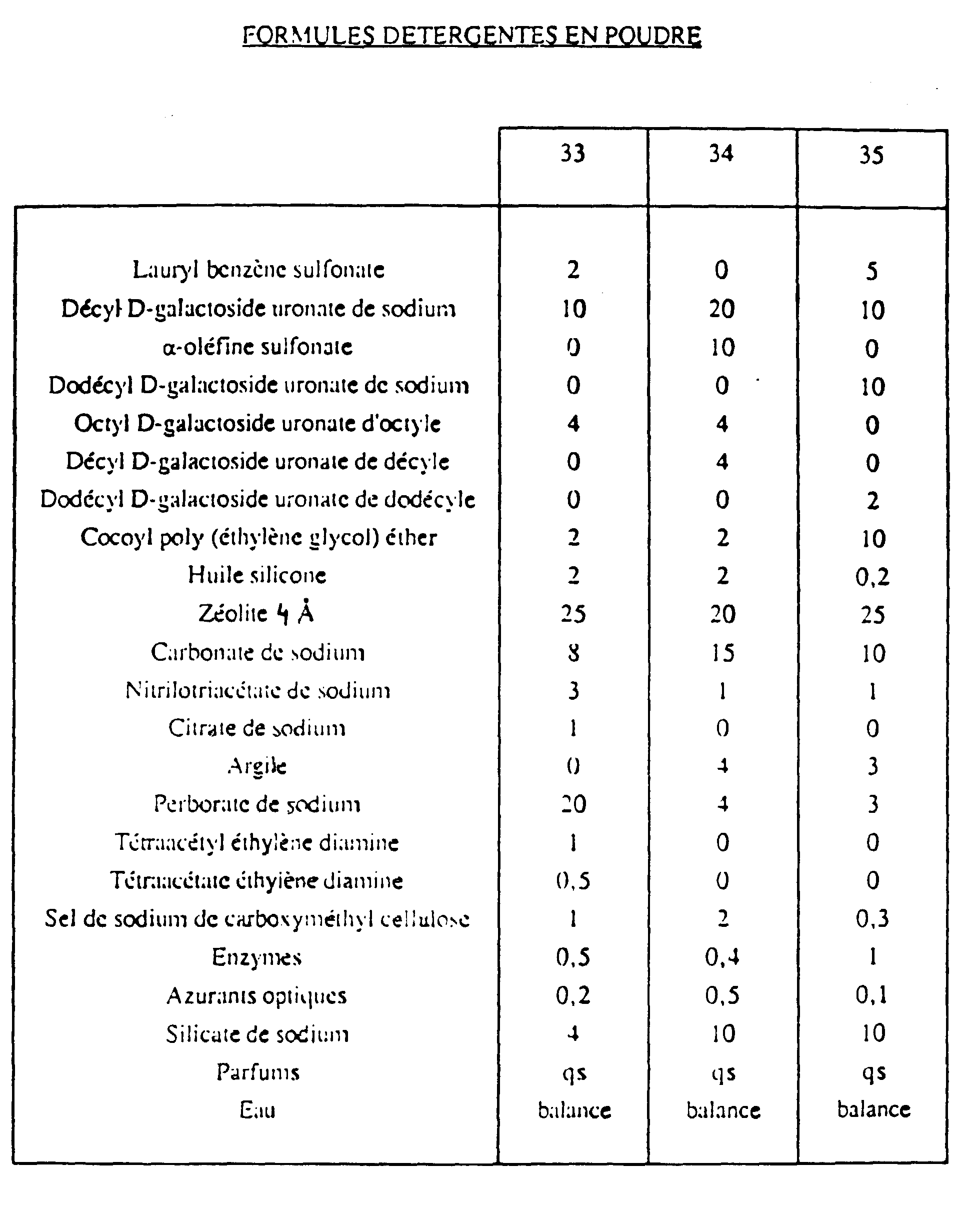

### EXEMPLES N° 28 A 30 :

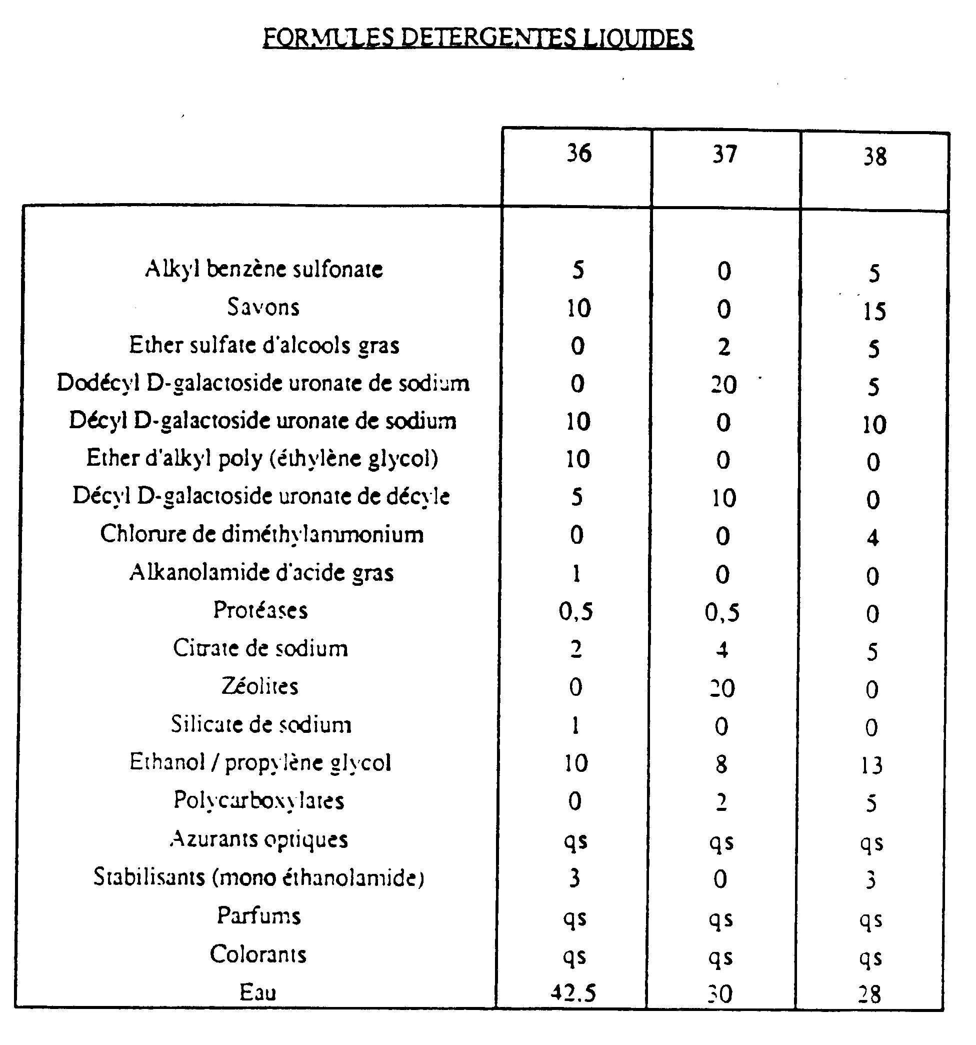

### EXEMPLES N° 31 A 34 :

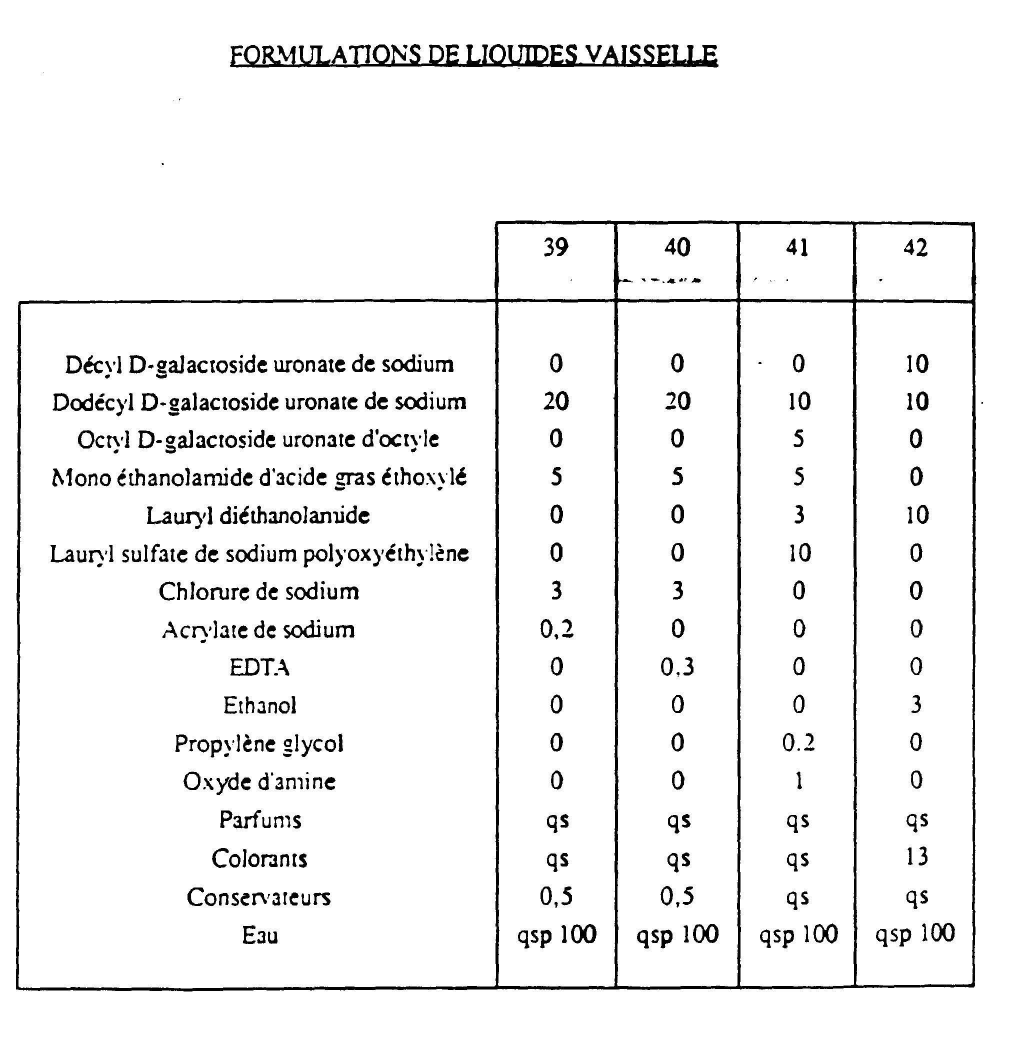

### EXEMPLES N° 35 A 38 :

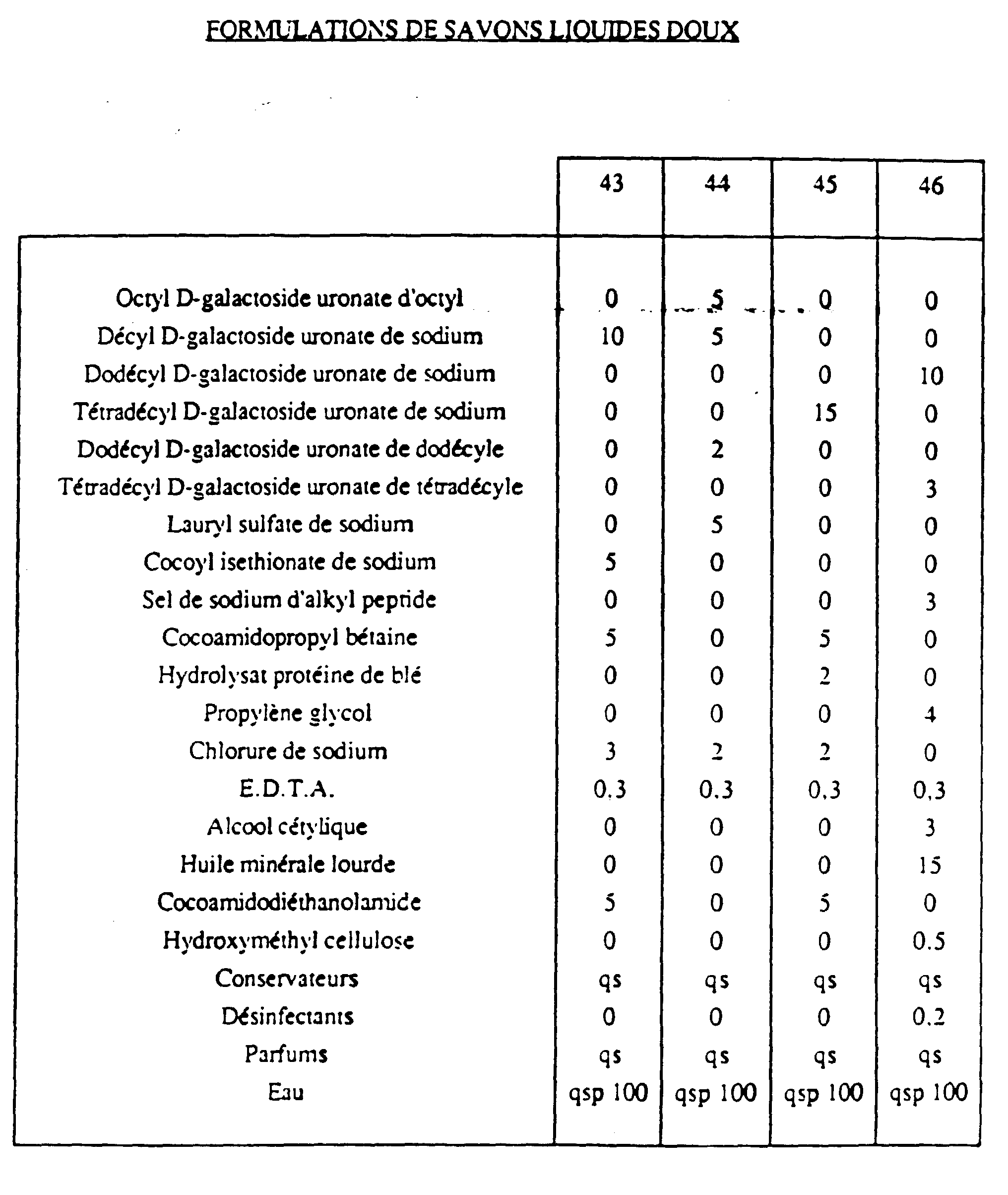

### EXEMPLES N° 39 A 42 :

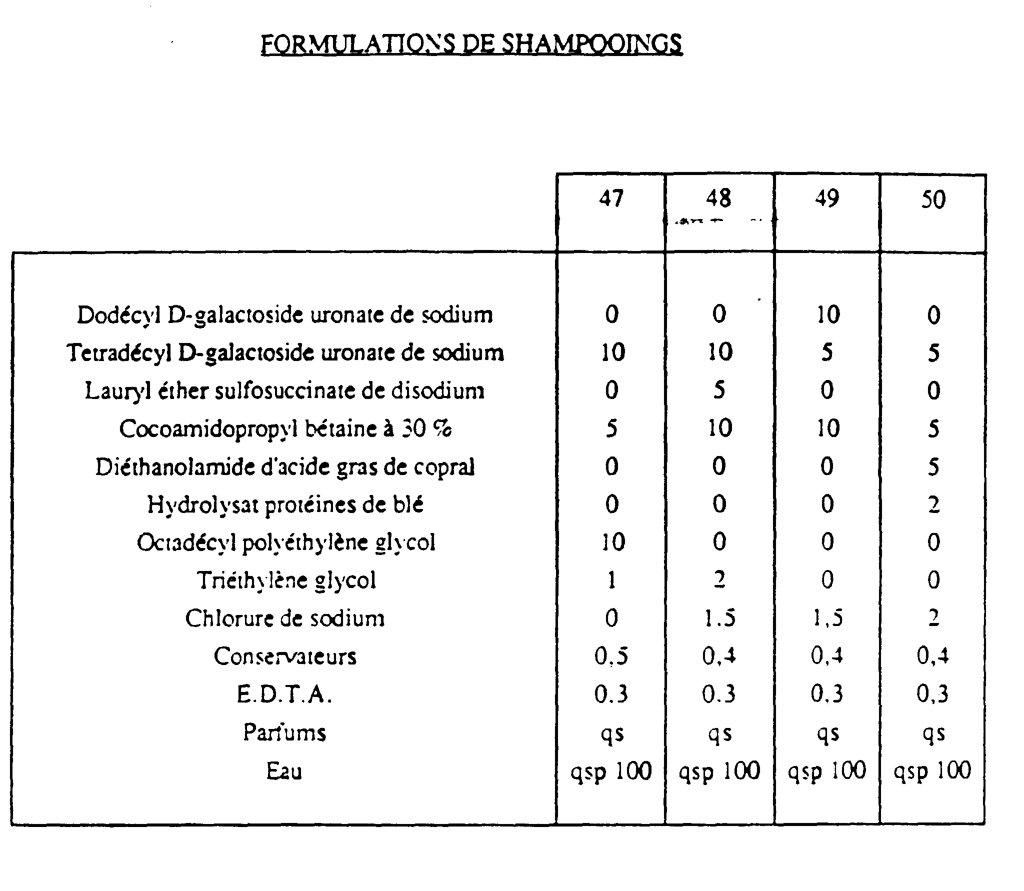

### EXEMPLES N° 43 A 44 :

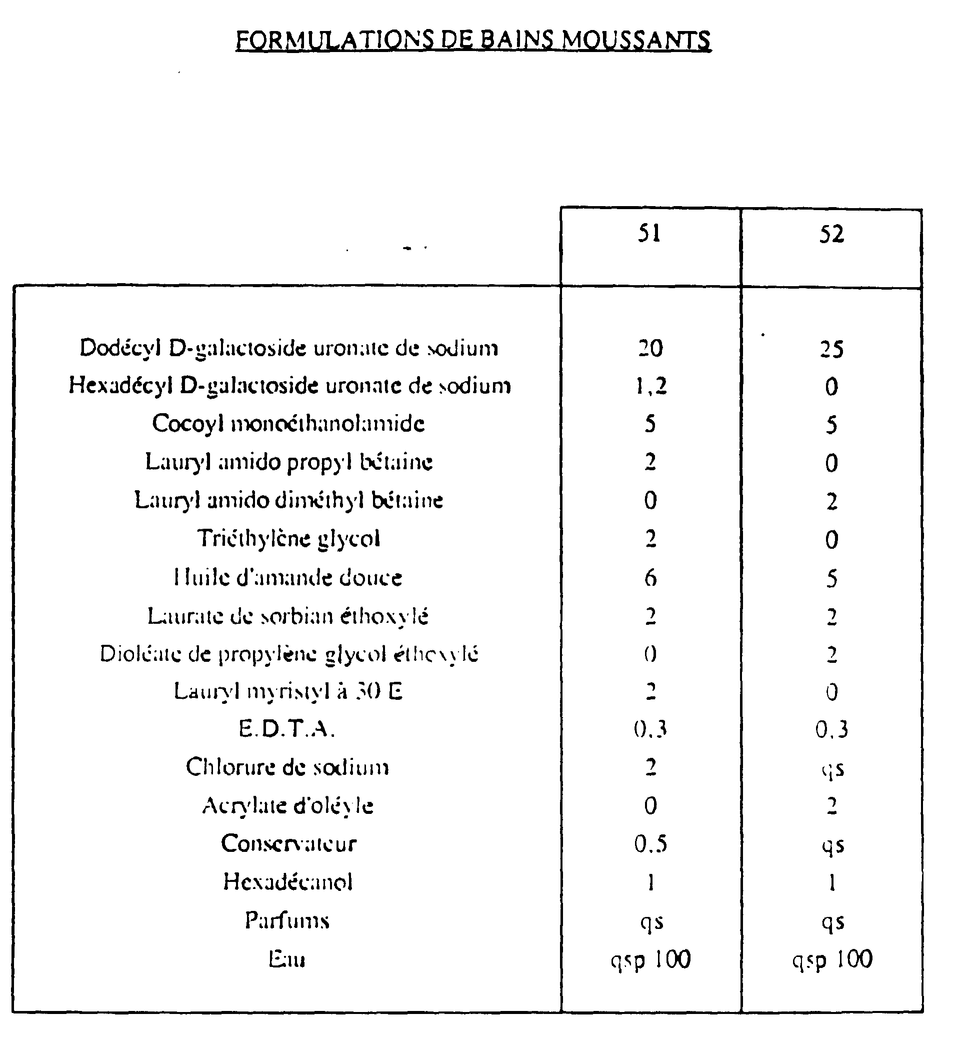

### EXEMPLES N° 45 A 46 :

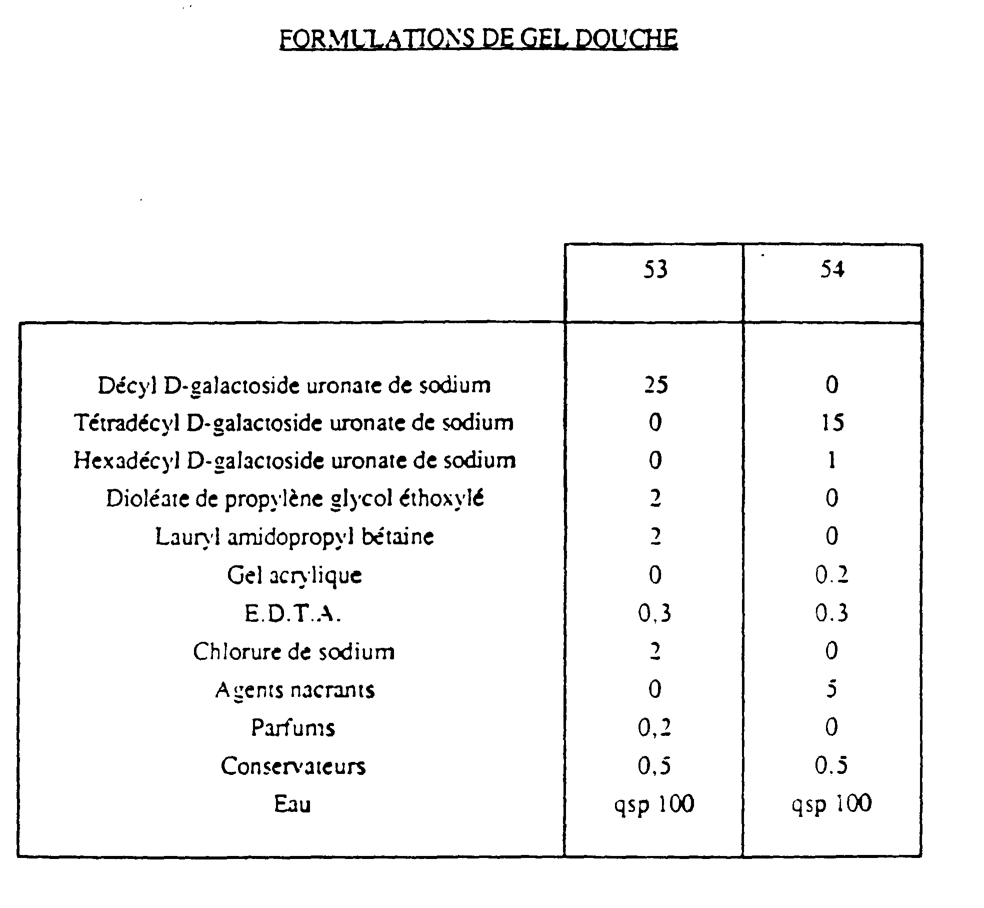

## Revendications (Revendications pour l'(les) Etat(s) contractant(s) suivant(s): AT, BE, CH, LI, DE, DK, FR, GB, GR, IT, LU, MC, NL, PT, SE)

1. Dérivé de l'acide galacturonique de formule: 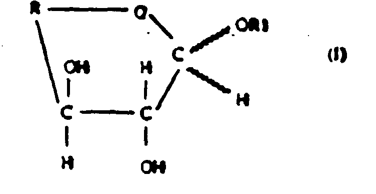
R1 étant alkyle linéaire ou ramifié ayant de 8 à 22 atomes de carbone,
R étant 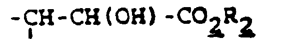 ou 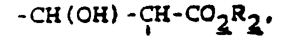 dont le carbone portant le groupe hydroxyle n'est pas relié à l'atome d'oxygène endocylique.
R2 étant l'hydrogène, un atome de métal alcalin, de métal alcalino-terreux, un groupe ammonium quaternaire de formule 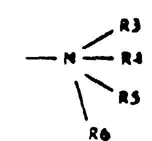 dans laquelle chacun de R3 à R6 est indépendamment des autres l'hydrogène, alkyle ayant de 1 à 6 atomes de carbone, ou hydroalkyle ayant de 1 à 6 atomes de carbone, à l'exception du palmityl-alpha-D-galacutronide.

2. Dérivé suivant la revendication 1, caractérisé en ce que R1 est alkyle ayant de 8 à 14 atomes de carbone.

3. Dérivé suivant la revendication 2, caractérisé en ce que R1 est décyle.

4. Dérivé suivant la revendication 1, caractérisé en ce que R1 est alkyle ayant de 14 à 22 atomes de carbone.

5. Procédé de préparation de dérivés de l'acide galacturonique de formule: 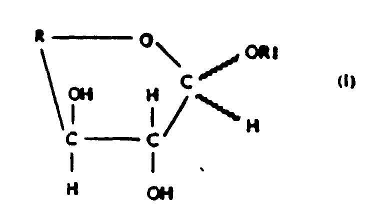
R1 étant alkyle linéaire ou ramifié ayant de 8 à 22 atomes de carbone,
R étant 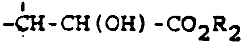 ou 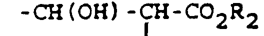 dont le carbone portant le groupe hydroxyle n'est pas relié à l'atome d'oxygène endocyclique,
R2 étant l'hydrogène, un atome de métal alcalin, de métal alcalino-terreux, un groupe ammonium quaternaire de formule 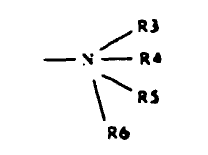
dans laquelle chacun de R3 à R6 est indépendamment des autres l'hydrogène, alkyle ayant de 1 à 6 atomes de carbone, ou hydroxyalkyle ayant de 1 à 6 atomes de carbone, caractérisé en ce qu'il consiste à faire réagir un alcool de formule R1OH sur l'acide galacturonique de formule: 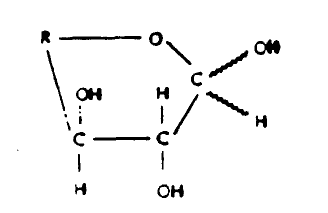 R étant 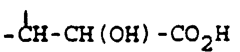 ou 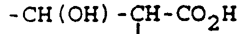 dont le carbone portant le groupe hydroxyle n'est pas relié à l'atome d'oxygène endocyclique pour obtenir un mélange d'alkylgalactoside uronates d'alkyle de formule 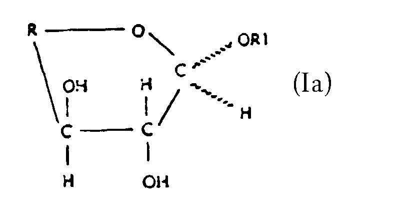 dans laquelle R2 est R1 et R a les deux significations indiquées, à séparer chaque alkylgalactoside uronate d'alkyle du mélange et pour obtenir les dérivés (I) dans lesquels R2 est un métal alcalin, un métal alcalino-terreux ou un groupe ammonium quaternaire, à salifier un dérivé de formule (la) par une base de formule Me(OH)x dans laquelle Me est un métal alcalin, un métal alcalino-terreux ou 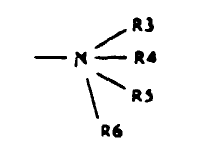 x étant la valence du métal ou étant égal à 1 quand Me est 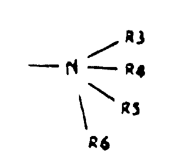 et pour obtenir les dérivés (I) dans lesquels R2 est l'hydrogène, à acidifier les dérivés (I) dans lesquels R2 est un métal ou un groupe ammonium quaternaire par un acide.

6. L'utilisation comme agent tensio-actif d'un dérivé de l'acide galacturonique de formule : 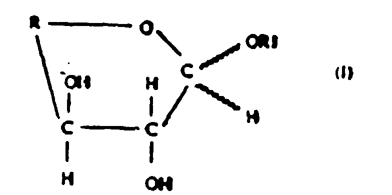
R1 étant alkyle linéaire ou ramifié ayant de 8 à 22 atomes de carbone,
R étant 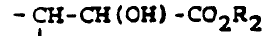 ou 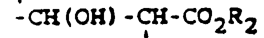 dont le carbone portant le groupe hydroxyle n'est pas relié à l'atome d'oxygène endocyclique,
R2 étant l'hydrogène, un atome de métal alcalin, de métal alcalino-terreux, un groupe ammonium quaternaire de formule 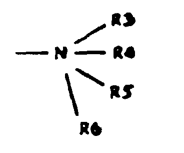 dans laquelle chacun de R3 à R6 est indépendamment des autres l'hydrogène, alkyle ayant de 1 à 6 atomes de carbone, ou hydroxyalkyle ayant de 1 à 6 atomes de carbone

7. L'utilisation d'un dérivé de l'acide galacturonique de formule (1) défini à la revendication 6 comme détergent.

8. L'utilisation selon la revendication 7, caractérisé en ce que R1 est alkyle ayant de 8 à 14 atomes de carbone.

9. L'utilisation d'un dérivé de l'acide galacturonique de formule (1) défini à la revendication 6 en cosmétologie.

## Revendications (Revendications pour l'(les) Etat(s) contractant(s) suivant(s): ES)

1. Procédé de préparation de dérivés de l'acide galacturonique de formule: 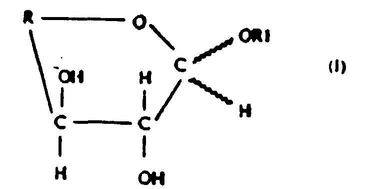
R1 étant alkyle linéaire ou ramifié ayant de 8 à 22 atomes de carbone,
R étant 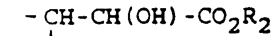 ou 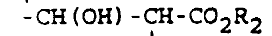 dont le carbone portant le groupe hydroxyle n'est pas relié à l'atome d'oxygène endocyclique,
R2 étant l'hydrogène, un atome de métal alcalin, de métal alcalino-terreux, un groupe ammonium quaternaire de formule 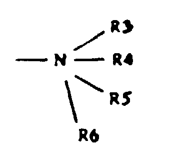 dans laquelle chacun de R3 à R6 est indépendamment des autres l'hydrogène, alkyle ayant de 1 à 6 atomes de carbone, ou hydroxyalkyle ayant de 1 à 6 atomes de carbone, caractérisé en ce qu'il consiste à faire réagir un alcool de formule RlOH sur l'acide galacturonique de formule: 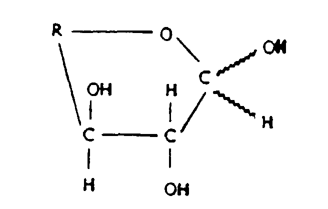 R étant 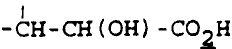 ou 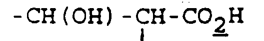 dont le carbone portant le groupe hydroxyle n'est pas relié à l'atome d'oxygène endocyclique pour obtenir un mélange d'alkylgalactoside uronates d'alkyle de formule 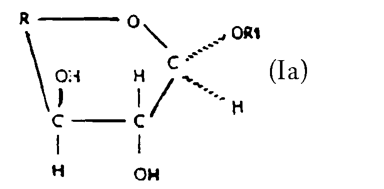 dans laquelle R2 est R1 et R a les deux significations indiquées et à séparer chaque alkylgalactoside uronate d'alkyle du mélange et, pour obtenir les dérivés (I) dans lesquels R2 est un métal alcalin, un métal alcalino-terreux ou un groupe ammonium quaternaire, à salifier un dérivé de formule (la) par une base de formule Me(OH)x dans laquelle Me est un métal alcalin, un métal alcalino-terreux ou 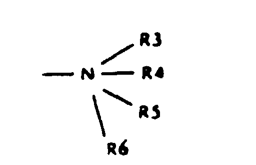 x étant la valence du métal ou étant égal à 1 quand Me est 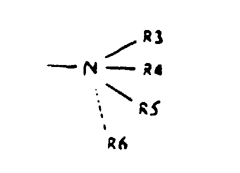 et pour obtenir les dérivés (I) dans lesquels R2 est l'hydrogène, à acidifier les dérivés (I) dans lesquels R2 est un métal ou un groupe ammonium quaternaire par un acide.

2. Procédé suivant la revendication 1, caractérisé en ce que R1 est alkyle ayant de 8 à 14 atomes de carbone.

3. Procédé suivant la revendication 2, caractérisé en ce que R1 est décyle.

4. Procédé suivant la revendication 1, caractérisé en ce que R1 est alkyle ayant de 14 à 22 atomes de carbone.

5. L'utilisation comme agent tensio-actif d'un dérivé de l'acide galacturonique de formule : 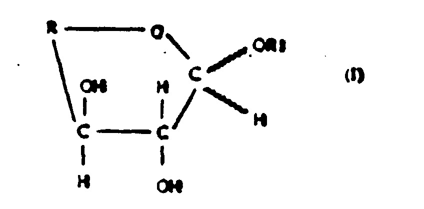
R1 étant alkyle linéaire ou ramifié ayant de 8 à 22 atomes de carbone, R étant 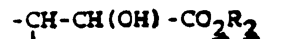 ou 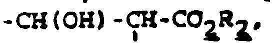 dont le carbone portant le groupe hydroxyle n'est pas relié à l'atome d'oxygène endocylique.
R2 étant l'hydrogène, un atome de métal alcalin, de métal alcalino-terreux, un groupe ammonium quaternaire de formule 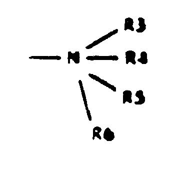 dans laquelle chacun de R3 à R6 est indépendamment des autres l'hydrogène, alkyle ayant de 1 à 6 atomes de carbone, ou hydroalkyle ayant de 1 à 6 atomes de carbone.

6. L'utilisation d'un dérivé de l'acide galacturonique de formule (1) défini à la revendication 5 comme détergent.

7. L'utilisation selon la revendication 6, caractérisée en ce que R1 est alkyle ayant de 8 à 14 atomes de carbone.

8. L'utilisation d'un dérivé de l'acide galacturonique de formule (1) défini à la revendication 5 en cosmétologie.

## Patentansprüche (Patentansprüche für folgende(n) Vertragsstaat(en): AT, BE, CH, LI, DE, DK, FR, GB, IT, LU, MC, NL, PT, SE)

1. Derivat der Galacturonsäure der Formel: 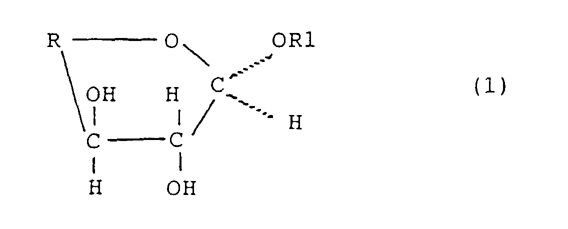 wobei:
R1 eine gerad- oder verzweigtkettige Alkylgruppe mit 8 - 22 Kohlenstoffatomen,
R 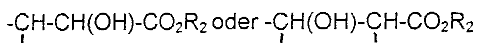 ist, wobei das Kohlenstoffatom, das die Hydroxylgruppe trägt, nicht mit dem endozyklischen Sauerstoffatom verbunden ist;
R2 Wasserstoff, ein Alkalimetall, ein Erdalkalimetall, eine quartäre Ammoniumgruppe der Formel: 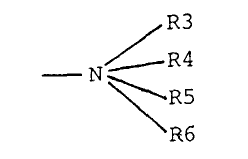 sein kann, wobei jeder der Reste R3 bis R6 unabhängig von den anderen Wasserstoff, eine Alkylgruppe mit 1 bis 6 Kohlenstoffatomen, oder eine Hydroxyalkylgruppe mit 1 bis 6 Kohlenstoffatomen sein kann, mit Ausnahme von Palmityl-Alpha-D-Galacturonid.

2. Derivat nach Anspruch 1, dadurch gekennzeichnet, daß R1 eine Alkylgruppe mit 8 bis 14 Kohlenstoffatomen ist.

3. Derivat nach Anspruch 2, dadurch gekennzeichnet, daß R1 eine Decylgruppe ist.

4. Derivat nach Anspruch 1, dadurch gekennzeichnet, daß R1 eine Alkylgruppe mit 14 bis 22 Kohlenstoffatomen ist.

5. Verfahren zur Herstellung der Derivate der Galacturonsäure der Formel: 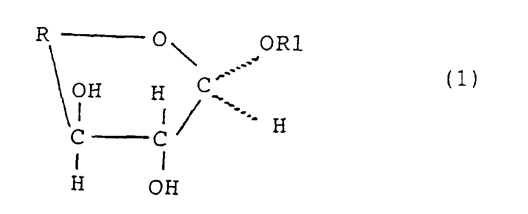 wobei
R1 eine lineare oder verzweigtkettige Alkylgruppe mit 8 bis 22 Kohlenstoffatomen;
R 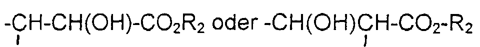 sein kann, wobei das die Hydroxylgruppe tragende Kohlenstoffatom nicht dem endozyklischen Sauerstoffatom verbunden ist,
R2 Wasserstoff, ein Alkalimetall, ein Erdalkalimetall, eine quartäre Ammoniumgruppe der Formel: 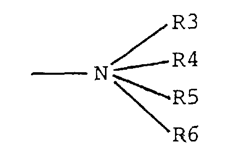 sein kann, wobei jeder der Reste R3 bis R6 unabhängig von den anderen Wasserstoff, eine Alkylgruppe mit 1 bis 6 Kohlenstoffatomen oder eine Hydroxyalkylgruppe mit 1 bis 6 Kohlenstoffatomen sein kann, dadurch gekennzeichnet, daß es daraus besteht, daß ein Alkohol der allgemeinen Formel R1OH mit der Galacturonsäure der Formel: 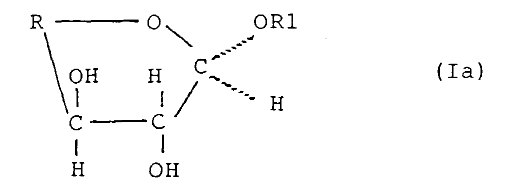 umgesetzt wird, wobei
R 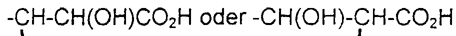 sein kann, wobei das die Hydroxylgruppe tragende Kohlenstoffatom nicht mit dem endozyklischen Wasserstoffatom verbunden ist, um eine Mischung von Alkylgalactosid und Alkyluronat der Formel 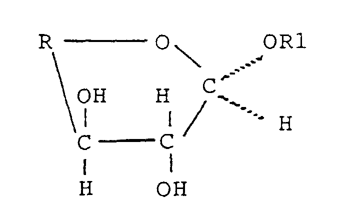 ist, wobei das R2, R1 oder R2 sein kann mit seinen angegebenen Bedeutungen, Abtrennen jeder Alkylgalactosidalkyluronsäure aus der Mischung, um die Derivate (I) zu erhalten, in denen R2 ein Alkalimetall, ein Erdalkalimetall oder eine quartäre Ammoniumgruppe ist und Aussalzen eines Derivats der Formal (la) durch eine Base der Formel Me(OH)x, wobei Me ein Alkalimetall oder ein Erdmetall oder 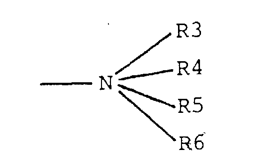 ist; wobei x die Valenz des Metalls oder gleich 1, wobei Me ist: 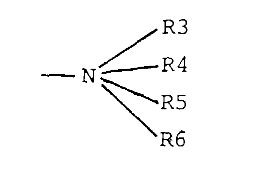 und Erhalt der Derivate (1), in denen R2 Wasserstoff ist, indem die Derivate (1), in denen R2 ein Metall oder eine quartäre Ammoniumgruppe ist, mit einer Säure angesäuert werden.

6. Verwendung eines Derivats der Galacturonsäure als Tensid der Formel: 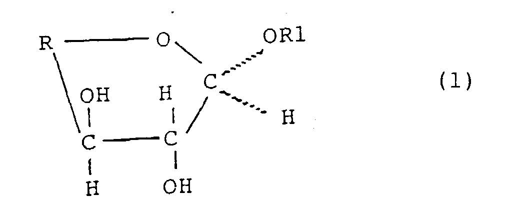
wobei R1 eine lineare oder eine verzweigtkettige Alkylgruppe mit 8 bis 22 Kohlenstoffatomen;
R 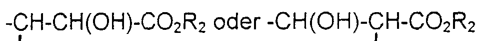 sein kann, wobei das die Hydroxylgruppe tragende Kohlenstoffatom nicht mit dem endozyklischen Sauerstoffatom verbunden ist,
R2 Wasserstoff, ein Alkalimetall, ein Erdalkalimetall oder ein quartäre Ammoniumgruppe der Formel: 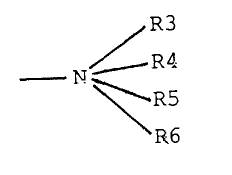 sein kann, wobei jeder Rest R3 bis R6 unabhängig von den anderen Wasserstoff, eine Akylgruppe mit 1 - 6 Kohlenstoffatomen oder Hydroxyalkylgruppe mit 1 bis 6 Kohlenstoffatomen sein kann.

7. Verwendung eines Derivats der Galacturonsäure der Formel (I) gemäß Anspruch 6 als Detergens.

8. Verwendung nach Anspruch 7, dadurch gekennzeichnet, daß R1 eine Alkylgruppe mit 8 bis 14 Kohlenstoffatomen ist.

9. Verwendung eines Derivats der Galacturonsäure der Formel (I) gemäß Anspruch 6 in der Kosmetologie.

## Patentansprüche (Patentansprüche für folgende(n) Vertragsstaat(en): ES)

1. Verfahren zur Herstellung eines Derivats der Galacturonsäure der Formel: 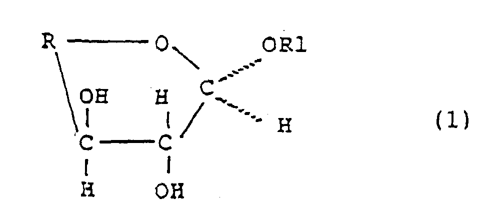 wobei
R1 eine gerad- oder verzweigtkettige Alkylgruppe mit 8 bis 22 Kohlenstoffatomen; und
R -CH-CH(OH)-CO₂R₂ oder -CH(OH)-CH-CO₂R₂ sein kann, wobei das die Hydroxylgruppe tragende Kohlenstoffatom nicht mit dem endozyklischen Sauerstoffatom verbunden ist,
R2 Wasserstoff, ein Alkalimetall, ein Erdalkalimetall, oder eine quartäre Ammoniumgruppe der Formel 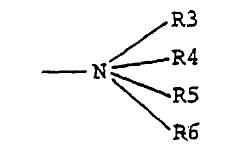 sein kann, wobei jeder Rest R 3 bis R6 unabhängig von den anderen Wasserstoff, eine Alkylgruppe mit 1 bis 6 Kohlenstoffatomen oder eine Hydroxyalkylgruppe mit 1 bis 6 Kohlenstoffatomen sein kann, dadurch gekennzeichnet, daß es daraus besteht, daß es einen Alkohol der Formel R1OH mit der Glacturonsäure der Formel: 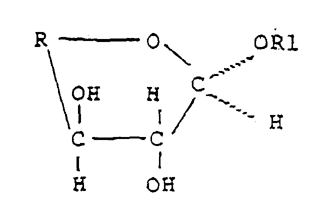 umsetzt, wobei R 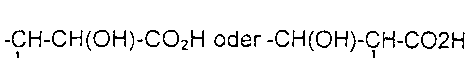 sein kann, wobei das die Hydroxylgruppe tragende Kohlenstoffatom nicht mit dem endozyklischen Sauerstoffatom verbunden ist, um eine Mischung von Alkylgalactosidalkyluronaten der Formel: 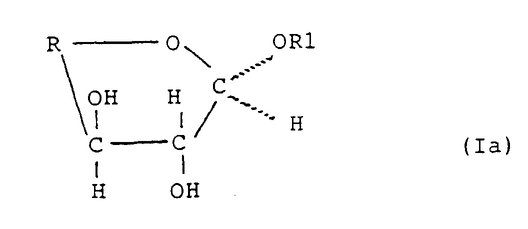
wobei R2 R1 ist und R die beiden oben angegebenen Bedeutungen haben kann, zu erhalten; und
Abtrennen jedes Alkyls derAlkylgalaktosituronsäure aus der Mischung und, um die Derivate (1), in denen R2 ein Alkalimetall, ein Erdalkalimetall oder eine quartäre Ammoniumgruppe ist, Aussalzen des Derivats der Formel (la) durch eine Base der Formel Me(OH)x wobei Me ein Alkalimetall, Erdalkalimetall oder 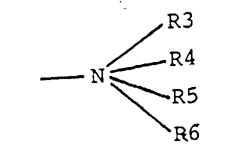 sein kann, wobei x die Valenz des Metalls oder gleich 1 ist, wobei Me ist: 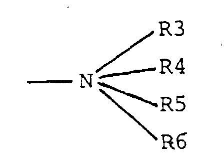 um die Derivate (I) zu erhalten, in denen R2 Wasserstoff ist, die Derivate (1), in denen R2 ein Metall oder eine quartäre Ammoniumgruppe ist, durch eine Säure ansäuert.

2. Verfahren nach Anspruch 1, dadurch gekennzeichnet, daß R1 eine Alkylgruppe mit 8 bis 14 Kohlenstoffatomen ist.

3. Verfahren nach Anspruch 2, dadurch gekennzeichnet, daß R1 eine Decylgruppe ist.

4. Verfahren nach Anspruch 1, dadurch gekennzeichnet, daß R1 eine Alkylgruppe mit 14 bis 22 Kohlenstoffatomen ist.

5. Verwendung eines Derivats der Galacturonsäure der Formel: 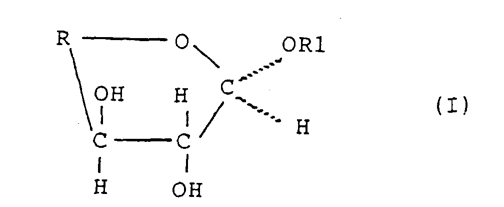 als Tensid, wobei R1 eine gerad- oder verzweigtkettige Alkylgruppe mit 8 bis 22 Kohlenstoffatomen, R 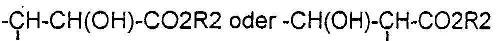 sein kann, wobei der die Hydroxylgruppe tragende Kohlenstoff nicht mit dem endozyklischen Sauerstoffatom verbunden ist; R2 Wasserstoff, ein Alkalimetall, ein Erdalkalimetall, oder eine quartäre Ammoniumgruppe der Formel: 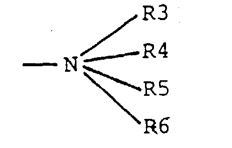 sein kann, wobei jeder R1 bis R6 unabhängig von den anderen Wasserstoff, eine Alkylgruppe mit 1 bis 6 Kohlenstoffatomen, oder eine Hydroxyalkylgruppe mit 1 bis 6 Kohlenstoffatomen sein kann.

6. Verwendung eines Derivats der Galacturonsäure der Formel (1), wie in Anspruch 5 definiert, als Detergens.

7. Verwendung nach Anspruch 6, dadurch gekennzeichnet, daß R1 eine Alkylgruppe mit 8 bis 14 Kohlenstoffatomen ist.

8. Verwendung eines Derivats der Galacturonsäure der Formel (1), wie sie in Anspruch 5 definiert ist, in der Kosmetologie.

## Claims (Claims for the following Contracting State(s): AT, BE, CH, LI, DE, DK, FR, GB, GR, IT, LU, MC, NL, PT, SE)

1. Galacturonic acid derivatives of formula: 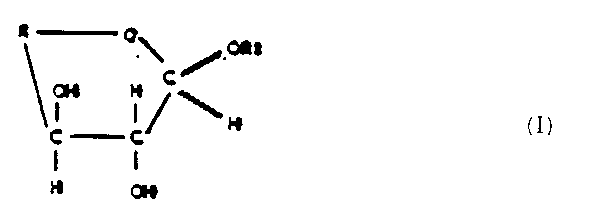
R1 being a linear or branched alkyl having 8 to 22 carbon atoms,
R being:
〉CH-CH(OH)-CO₂R₁
or 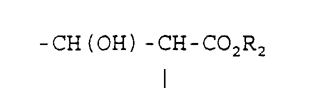 for which the carbon carrying the hydroxyl group is not attached to the endocyclic oxygen atom,
R₂ is hydrogen, an alkali-metal atom, an alkaline-earth metal atom, a quaternary ammonium group of formula 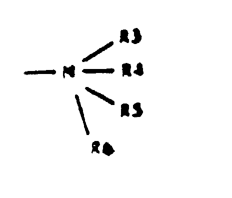 in which each of R3 to R6 is independently from the others hydrogen, an alkyl having 1 to 6 carbon atoms, or a hydroxyalkyl group having 1 to 6 carbon atoms, with the exception of palmityl-alpha-D-galacturonide.

2. Derivative according to claim 1, characterized in that R1 is an alkyl having 8 to 14 carbon atoms.

3. Derivative according to claim 2, characterized in that R1 is decyl.

4. Derivative according to claim 1, characterized in that R1 is an alkyl having 14 to 22 carbon atoms.

5. Method for preparing galacturonic acid derivatives of formula: 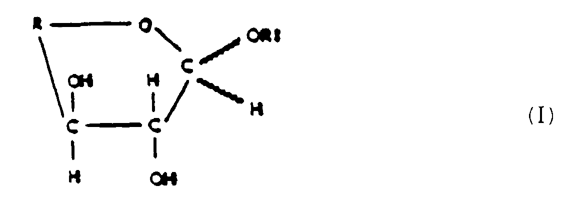
R1 being a linear or branched alkyl having 8 to 22 carbon atoms,
R being: 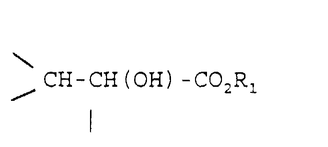 or 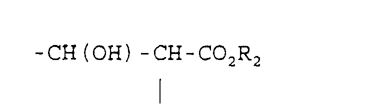 for which the carbon carrying the hydroxyl group is not attached to the endocyclic oxygen atom,
R₂ is hydrogen, an alkali-metal atom, an alkaline-earth metal atom, a quaternary ammonium group of formula 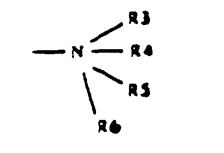 in which each of R3 to R6 is independently from the others hydrogen, an alkyl having 1 to 6 carbon atoms, or a hydroxyalkyl group having 1 to 6 carbon atoms, characterized in that it comprises reacting an alcohol of formula R1OH with the galacturonic acid of formula 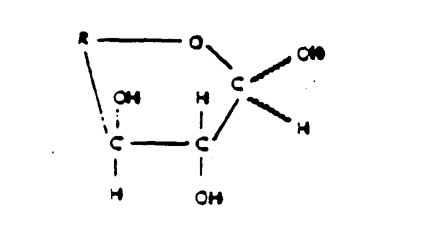 R being 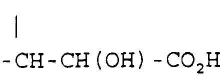 or 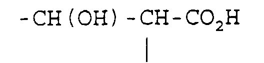 for which the carbon carrying the hydroxyl group is not attached to the endocyclic oxygen atom to give a mixture of alkyl alkylgalactoside uronates of formula 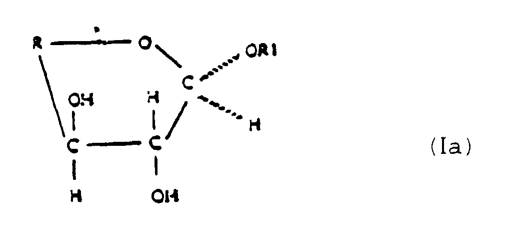 in which R2 is R1 and R has the two recited meanings, and separating each alkyl alkyl galactoside uronate from the mixture and for preparing the derivatives (I) in which R2 is an alkali metal, an alkaline-earth metal or a quaternary ammonium group salifying a derivative of formula (Ia) with a base of formula Me(OH)x in which Me is an alkali metal, an alkaline-earth metal or 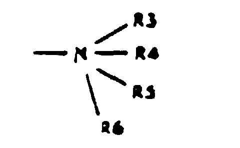 x being the valence of the metal or being 1 when Me is 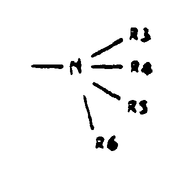 and for preparing the derivatives (I) in which R2 is hydrogen, acidifying the derivatives (I) in which R2 is a metal or a quaternary ammonium group with an acid.

6. The use as a surfactant of a galacturonic acid derivative of formula: 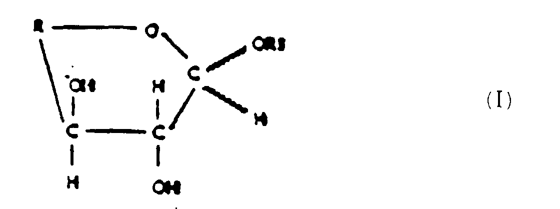
R1 being a linear or branched alkyl having 8 to 22 carbon atoms,
R being: 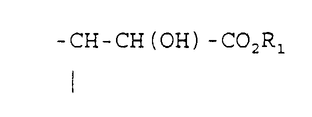 or 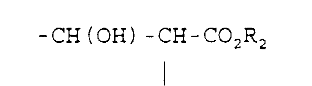 for which the carbon carrying the hydroxyl group is not attached to the endocyclic oxygen atom,
R₂ is hydrogen, an alkali-metal atom, an alkaline-earth metal atom, a quaternary ammonium group of formula 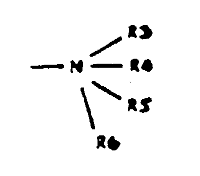 in which each of R3 to R6 is independently from the others hydrogen, an alkyl having 1 to 6 carbon atoms, or a hydroxyalkyl group having 1 to 6 carbon atoms.

7. The use of a derivative as defined in claim 6 as a detergent.

8. The use according to claim 7, characterized in that R1 is alkyl having 8 to 14 carbon atoms.

9. The use of a galacturonic acid derivative of formula (I) as defined in claim 6 in cosmetology.

## Claims (Claims for the following Contracting State(s): ES)

1. Method for preparing galacturonic acid derivatives of formula: 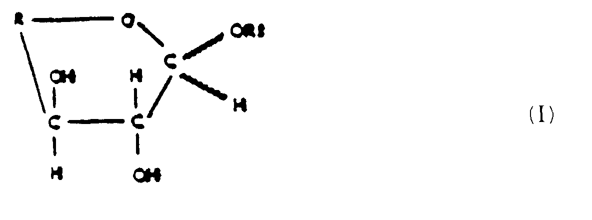
R1 being a linear or branched alkyl having 8 to 22 carbon atoms,
R being: 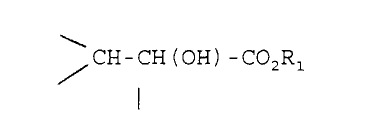 or 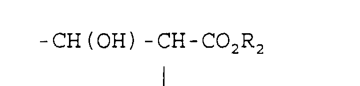 for which the carbon carrying the hydroxyl group is not attached to the endocyclic oxygen atom,
R₂ is hydrogen, an alkali-metal atom, an alkaline-earth metal atom, a quaternary ammonium group of formula 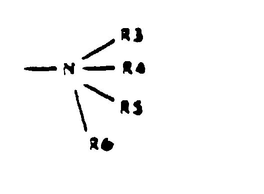 in which each of R3 to R6 is independently from the others hydrogen, an alkyl having 1 to 6 carbon atoms, or a hydroxyalkyl group having 1 to 6 carbon atoms, characterized in that it comprises reacting an alcohol of formula R1OH with the galacturonic acid of formula 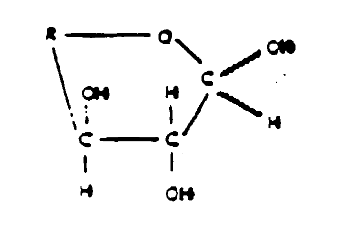
R being 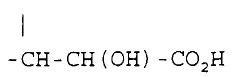 or 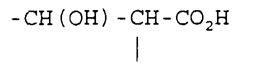 for which the carbon carrying the hydroxyl group is not attached to the endocyclic oxygen atom to give a mixture of alkyl alkylgalactoside uronates of formula 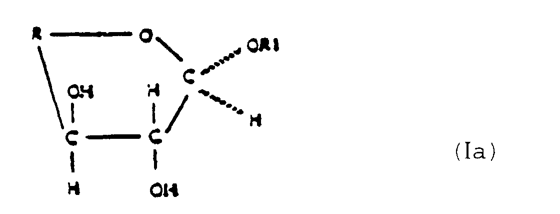 in which R2 is R1 and R has the two recited meanings, and separating each alkyl alkyl galactoside uronate from the mixture and for preparing the derivatives (I) in which R2 is an alkali metal, an alkaline-earth metal or a quaternary ammonium group salifying a derivative of formula (Ia) with a base of formula Me(OH)x in which Me is an alkali metal, an alkaline-earth metal or 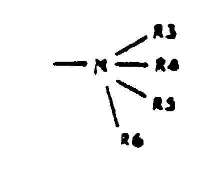 x being the valence of the metal or being 1 when Me is 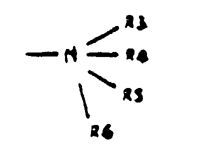 and for preparing the derivatives (I) in which R2 is hydrogen, acidifying the derivatives (I) in which R2 is a metal or a quaternary ammonium group with an acid.

2. Method according to claim 1, characterized in that R1 is an alkyl having 8 to 14 carbon atoms.

3. Method according to claim 2, characterized in that R1 is decyl.

4. Method according to claim 1, characterized in that R1 is an alkyl having 14 to 22 carbon atoms.

5. The use as a surfactant of a galacturonic acid derivative of formula: 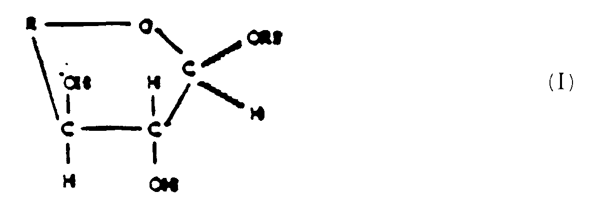
R1 being a linear or branched alkyl having 8 to 22 carbon atoms,
R being: 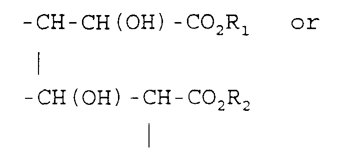 for which the carbon carrying the hydroxyl group is not attached to the endocyclic oxygen atom,
R₂ is hydrogen, an alkali-metal atom, an alkaline-earth metal atom, a quaternary ammonium group of formula 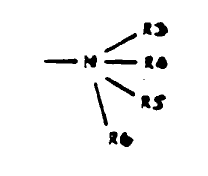 in which each of R3 to R6 is independently from the others hydrogen, an alkyl having 1 to 6 carbon atoms, or a hydroxyalkyl group having 1 to 6 carbon atoms.

6. The use of a derivative of a galacturonic acid as defined in claim 5 as a detergent.

7. The use according to claim 6, characterized in that R1 is alkyl having 8 to 14 carbon atoms.

8. The use of a galacturonic acid derivative of formula (I) as defined in claim 5 in cosmetology.
